# EUROPEAN PATENT SPECIFICATION

(11) **EP 4 238 484 B1**
(45) Date of publication and mention of the grant of the patent: **16.09.2026**
(21) Application number: 21885560.9
(22) Date of filing: 02.02.2021
(51) Int. Cl.: A61B 3/15, A61B 3/13, A61B 3/14, A61B 3/10, A61B 3/00

(54) **OPHTHALMOLOGICAL OBSERVATION DEVICE, METHOD FOR CONTROLLING SAME, PROGRAM, AND STORAGE MEDIUM**
OPHTHALMOLOGISCHE BEOBACHTUNGSVORRICHTUNG, VERFAHREN ZUR STEUERUNG DAVON, PROGRAMM UND SPEICHERMEDIUM
DISPOSITIF D'OBSERVATION OPHTALMOLOGIQUE, SON PROCÉDÉ DE COMMANDE, PROGRAMME, ET SUPPORT D'ENREGISTREMENT

(30) Priority: 27.10.2020 US 202063106087 P
(43) Date of publication of application: 06.09.2023
(73) Proprietor: TOPCON CORPORATION, Tokyo 174-8580 (JP)
(72) Inventor: YAMADA, Kazuhiro, Tokyo 174-8580 (JP); OOMORI, Kazuhiro, Tokyo 174-8580 (JP); FUKUMA, Yasufumi, Tokyo 174-8580 (JP)
(74) Representative: Gagel, Roland
(86) International application number: PCT/JP2021/003639
(87) International publication number: WO 2022/091435

(56) References cited:
- WO-A1-2016/061454
- WO-A1-2018/207466
- WO-A1-2020/179588
- JP-A- 2014 534 011
- US-A1- 2011 292 340
- US-A1- 2013 135 582
- US-A1- 2017 281 405
- US-A1- 2018 049 840
- US-A1- 2020 146 885
- US-B2- 10 251 783

## Description

### TECHNICAL FIELD

The present disclosure relates generally to an ophthalmic observation apparatus, a method of controlling the same, and a recording medium.

### BACKGROUND OF THE INVENTION

An ophthalmic observation apparatus is an apparatus for observing an eye of a patient (which will be referred to as a subject's eye hereinafter). Ophthalmic observation is conducted to grasp the condition of the subject's eye in various situations such as examination, surgery, and treatment.

Conventional ophthalmic observation apparatuses are configured to provide a user with a magnified image formed by an objective lens, a variable magnification optical system, etc. via an eyepiece. In recent years, some ophthalmic observation apparatuses are configured to photograph a magnified image formed by an objective lens, a variable magnification optical system, etc. with an image sensor, and display the photographed image obtained (such an ophthalmic observation apparatus will be referred to as a digital ophthalmic observation apparatus). Examples of such digital ophthalmic observation apparatuses include surgical microscopes, slit lamp microscopes, and fundus cameras (retinal cameras). In addition, various kinds of ophthalmic examination apparatuses such as refractometers, keratometers, tonometers, specular microscopes, wavefront analyzers, and microperimeters are also provided with the function of the digital ophthalmic observation apparatus.

Furthermore, some ophthalmic observation apparatuses of recent years use optical scanning (scanning-type ophthalmic observation apparatus). Examples of such ophthalmic observation apparatuses include scanning laser ophthalmoscopes (SLOs), and optical coherence tomography (OCT) apparatuses.

Generally, an ophthalmic observation apparatus is configured to provide a moving image of a subject's eye to a user (e.g., a health professional (health care practitioner) such as a doctor). A typical digital ophthalmic observation apparatus is configured to perform photographing of a moving image using infrared light and/or visible light as illumination light, and real-time display of the moving image obtained by the moving image photography. On the other hand, a typical scanning-type ophthalmic observation apparatus is configured to perform data collection (data acquisition) by repetitive optical scanning, real-time image reconstruction based on datasets sequentially collected, and real-time moving image display of images sequentially reconstructed. The real-time moving image provided in these ways is referred to as an observation image or a live image.

An ophthalmic observation apparatus capable of providing a real-time moving image is also used in surgery. There are various types of ophthalmic surgery and many ophthalmic surgeries are performed by inserting instruments into the eye. Instrument insertion requires an incision of an ocular tissue. For example, cataract surgery to replace the crystalline lens with an intraocular lens (IOL) typically involves corneal incision (keratotomy, sclerocorneal incision), crystalline lens anterior capsule incision (incision of the anterior capsule of the crystalline lens, referred to as capsulotomy, anterior capsulectomy, or the like) (e.g., Continuous Curvilinear Capsulorhexis (CCC)), and so forth. Further, in refractive surgery to implant an intraocular contact lens (ICL; also known as a phakic IOL) in the anterior chamber, corneal incision is usually made. In addition, minimally invasive glaucoma surgery (MIGS) involves incision of the trabecular meshwork and insertion of a stent into the trabecular meshwork. In vitreoretinal surgery (vitrectomy), in which a light guide (illumination) and various kinds of instruments are used, conjunctival incision and sclerotomy (scleral incision) are generally made.

Such incisions in ocular tissue need to be made at appropriate locations. However, it is difficult to determine an appropriate incision target position since there are few landmarks in the eye, making a mark on the eye is difficult, and the eye may move during surgery. The same applies to treatments or procedures other than incisions.

PATENT DOCUMENT 1: Japanese Unexamined Patent Application Publication No. 2019-162336

US 2017/281405 A1 describes an ophthalmic treatment device and a control method therefor. The ophthalmic treatment device comprises a treatment beam radiation unit which radiates a treatment beam to a fundus, a guide image unit which obtains a guide image of an area belonging to an area of the fundus and comprising a position where the treatment beam has been radiated, and a display unit which displays a fundus image of a patient and the position where the treatment beam has been radiated in the fundus image using the guide image.

WO 2020/179588 A1 discloses a surgical microscope system comprising a microscope optical system, an imaging device that captures an image of an eye as an operation subject through the microscope optical system, and an image processing device that magnifies a first region in the image and reduces the size of a second region in the image so that the second region can be displayed in a region other than the magnification region.

US 2020/146885 A1 discloses an image processing with an image recognition section that performs an image recognition with respect to a front image of an eye, a display information generator that generates display information and a controller that controls at least one of a cross-sectional information acquisition section or the display information generator, the cross-sectional information acquisition section acquiring cross-sectional information regarding a cross section of an eye.

JP 2014 534011 A describes a method for treating the retina of a patient's eye by projecting a first beam onto retinal tissue of the eye, defining, via the first beam, a boundary separating the retinal tissue to a first area and a second area, and delivering, via a second beam, a therapeutic treatment to the retinal tissue of the eye by selectively directing the second beam onto the retinal tissue within the first area.

WO 2016/061454 A1 describes a method of cataract surgery in an eye of a patient by identifying a feature selected from the group consisting of an axis, a meridian, and a structure of an eye by corneal topography, and forming fiducial mark incisions with a laser beam along the axis, meridian or structure in the cornea outside the optical zone of the eye.

US 2011/292340 A1 dislcoses an ophthalmic apparatus for guiding positioning an intraocular lens for astigmatism correction. The apparatus includes an obtaining unit for obtaining an anterior segment image of a patient's eye and an astigmatic axis of a patient's cornea, a feature point designating unit for defining a feature point of an iris or a sclera on the anterior segment image, and a display control unit for superimposing and displaying a gauge image that models a cornea gauge and an axis that represents the astigmatic axis of the cornea on the anterior segment image.

### BRIEF SUMMARY OF THE INVENTION

One object of the present disclosure is to provide a novel method or technique for facilitating ophthalmic observation.

The object is achieved with the ophthalmic observation apparatus according to claim 1, the method of controlling an ophthalmic observation apparatus according to claim 3, and the computer-readable non-transitory recording medium according to claim 4.

The proposed apparatus and method allow ophthalmic surgery to be facilitated.

### BRIEF DESCRIPTION OF THE SEVERAL VIEWS OF THE DRAWING

FIG. 1 is a diagram illustrating an example of a configuration of an ophthalmic observation apparatus (ophthalmic surgical microscope) according to an embodiment example.
FIG. 2 is a diagram illustrating an example of a configuration of an ophthalmic observation apparatus according to an embodiment example.
FIG. 3 is a diagram illustrating an example of a configuration of an ophthalmic observation apparatus according to an embodiment example.
FIG. 4 is a diagram illustrating an example of a configuration of an ophthalmic observation apparatus according to an embodiment example.
FIG. 5 is a diagram illustrating an example of a configuration of an ophthalmic observation apparatus according to an embodiment example.
FIG. 6 is a diagram illustrating an example of a configuration of an ophthalmic observation apparatus according to an embodiment example.
FIG. 7 is a diagram illustrating an example of a configuration of an ophthalmic observation apparatus according to an embodiment example.
FIG. 8A is a flowchart illustrating an example of processing performed by an ophthalmic observation apparatus according to an embodiment example.
FIG. 8B is a flowchart illustrating an example of processing performed by an ophthalmic observation apparatus according to an embodiment example.
FIG. 9A is a diagram for describing an example of processing performed by an ophthalmic observation apparatus according to an embodiment example.
FIG. 9B is a diagram for describing an example of processing performed by an ophthalmic observation apparatus according to an embodiment example.
FIG. 9C is a diagram for describing an example of processing performed by an ophthalmic observation apparatus according to an embodiment example.
FIG. 9D is a diagram for describing an example of processing performed by an ophthalmic observation apparatus according to an embodiment example.
FIG. 9E is a diagram for describing an example of processing performed by an ophthalmic observation apparatus according to an embodiment example.
FIG. 10A is a diagram for describing an example of processing performed by an ophthalmic observation apparatus according to an embodiment example.
FIG. 10B is a diagram for describing an example of processing performed by an ophthalmic observation apparatus according to an embodiment example.
FIG. 10C is a diagram for describing an example of processing performed by an ophthalmic observation apparatus according to an embodiment example.
FIG. 11 is a diagram for describing an example of processing performed by an ophthalmic observation apparatus according to an embodiment example.

### DETAILED DESCRIPTION OF THE INVENTION

Some aspect examples of an ophthalmic observation apparatus, a method of controlling the same, a program, and a recording medium according to some embodiments will be described in detail with reference to the drawings. It should be noted that any of the matters and items described in the documents cited in the present disclosure and any known techniques and technologies may be combined with any of the aspect examples.

The ophthalmic observation apparatus according to some aspect examples is used in medical practice (healthcare practice) such as surgery, examination, and treatment, in order to grasp (understand, recognize, find) the state of the subject's eye. The ophthalmic observation apparatus of the aspect examples described herein is mainly a surgical microscope system. However, ophthalmic observation apparatuses of embodiments are not limited to surgical microscope systems. For example, the ophthalmic observation apparatus of some aspect examples may be any of a slit lamp microscope, a fundus camera, a refractometer, a keratometer, a tonometer, a specular microscope, a wavefront analyzer, a microperimeter, an SLO, and an OCT apparatus. Also, the ophthalmic observation apparatus of some aspect examples may be a system that includes any one or more of these apparatus examples. In a wider sense, the ophthalmic observation apparatus of some aspect examples may be any type of ophthalmic apparatus having an observation function.

A target ocular site for observation (ocular site to be observed, ocular site subject to observation) by using the ophthalmic observation apparatus may be any site of the subject's eye, and may be any site of the anterior eye segment and/or any site of the posterior eye segment. Examples of the observation target sites of the anterior eye segment include cornea, iris, anterior chamber, corner angle, crystalline lens, ciliary body, and zonule of Zinn. Examples of the observation target sites of the posterior eye segment include retina, choroid, sclera, and vitreous body. The observation target site is not limited to tissues of an eye ball, and may be any site subject to be observed in ophthalmic medical practice (and/or medical practice in other medical fields) such as eyelid, meibomian gland, and orbit (eye socket, eye pit).

The ophthalmic observation apparatus is used, for example, in surgery in which an artificial object is inserted into the eye. The artificial object may be a freely selected instrument (device) that is implanted (placed) in the eye. Examples of such an artificial object include an intraocular lens, an intraocular contact lens, and an MIGS device (stent). Note that the artificial objects may be a freely selected medical instrument such as a light guide, a surgical instrument, an examination instrument, or a treatment instrument.

At least one or more of the functions of the elements described in the present disclosure are implemented by using a circuit configuration (or circuitry) or a processing circuit configuration (or processing circuitry). The circuitry or the processing circuitry includes any of the followings, all of which are configured and/or programmed to execute at least one or more functions disclosed herein: a general purpose processor, a dedicated processor, an integrated circuit, a central processing unit (CPU), a graphics processing unit (GPU), an application specific integrated circuit (ASIC), a programmable logic device (e.g., a simple programmable logic device (SPLD), a complex programmable logic device (CPLD), or a field programmable gate array (FPGA)), a conventional circuit configuration or circuitry, and any combination of these. A processor is considered to be processing circuitry or circuitry that includes a transistor and/or another circuitry. In the present disclosure, circuitry, a unit, a means, or a term similar to these is hardware that executes at least one or more functions disclosed herein, or hardware that is programmed to execute at least one or more functions disclosed herein. Hardware may be the hardware disclosed herein, or alternatively, known hardware that is programmed and/or configured to execute at least one or more functions described herein. In the case where the hardware is a processor, which may be considered as a certain type of circuitry, then circuitry, a unit, a means, or a term similar to these is a combination of hardware and software. In this case, the software is used to configure the hardware and/or the processor.

### < Ophthalmic observation apparatus >

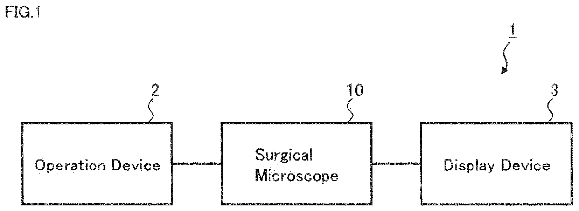

FIG. 1 shows the configuration of the ophthalmic observation apparatus of some aspect examples.

The ophthalmic observation apparatus 1 (surgical microscope system, operation microscope system) according to the present embodiment may include the operation device 2, the display device 3, and the surgical microscope (operation microscope) 10. In some aspects, the surgical microscope 10 may include at least one of the operation device 2 and the display device 3. In some aspects, the display device 3 may not be included in the ophthalmic observation apparatus 1. In other words, the display device 3 may be a peripheral device of the ophthalmic observation apparatus 1.

### < Operation device 2 >

The operation device 2 includes an operation device and/or an input device. For example, the operation device 2 may include any of a button, a switch, a mouse, a keyboard, a trackball, an operation panel, a dial, and so forth. Typically, the operation device 2 includes a foot switch, like standard (general, normal, usual) ophthalmic surgical microscopes. Further, the operation device 2 may also be configured in such a manner that the user performs operations using voice recognition, line-of-sight (gaze) input, or like input technologies.

### < Display device 3 >

The display device 3 displays an image of the subject's eye acquired by the surgical microscope 10. The display device 3 includes a display device such as a flat panel display. The display device 3 may include any of various kinds of display devices such as a touch panel. The display device 3 of some typical aspects includes a display device with a large screen. The display device 3 includes one or more display devices. In the case where the display device 3 includes two or more display devices, for example, one may be a display device with a relatively large screen and another one of the other(s) may be a display device with a relatively small screen. Also, a configuration may be employed in which a plurality of display regions is provided in one display device to display a plurality of pieces of information.

The operation device 2 and the display device 3 do not have to be separate devices. For example, a device having both the operation function and the display function, such as a touch panel, may be used as the display device 3. In such a case, the operation device 2 may include a computer program in addition to the touch panel. A content of an operation made by the operation device 2 is sent to a processor (not shown in the drawings) as an electric signal. Further, a graphical user interface (GUI) displayed on the display device 3 and the operation device 2 may be used to conduct operations (instructions) and input information. In some aspects, the functions of the operation device 2 and the display device 3 may be implemented with a touch screen.

### < Surgical microscope 10 >

The surgical microscope 10 is used for observation of the eye of a patient (subject's eye) in the supine position. The surgical microscope 10 performs photographing of the subject's eye to generate digital image data. In particular, the surgical microscope 10 generates a moving image of the subject's eye. The moving image (video, movie) generated by the surgical microscope 10 is transmitted to the display device 3 through a wired and/or wireless signal path and displayed on the display device 3. The user (e.g., surgeon) can carry out surgery while observing the subject's eye through the displayed image. In addition to such observation through the displayed image, the surgical microscope 10 of some aspects may also be capable of providing observation through an eyepiece as in conventional technology.

In some aspects, the surgical microscope 10 includes a communication device for transmitting and receiving electrical signals to and from the operation device 2. The operation device 2 receives an operation (instruction) performed by the user and generates an electric signal (operation signal) corresponding to the operation. The operation signal is transmitted to the surgical microscope 10 through a wired and/or wireless signal path. The surgical microscope 10 executes processing corresponding to the operation signal received.

### < Optical system of surgical microscope 10 >

An example of the configuration of the optical system of the surgical microscope 10 will be described. Below, directions are defined as follows, for convenience of description: the z direction is defined to be the optical axis direction (direction along the optical axis) of the objective lens (the z direction is, for example, the vertical direction, the up and down direction during surgery); the x direction is defined to be a predetermined direction perpendicular to the z direction (the x direction is, for example, the horizontal direction during surgery, and the left and right direction for the surgeon and the patient during surgery); and the y direction is defined to be the direction perpendicular to both the z and x directions (the y direction is, for example, the horizontal direction during surgery, the front and back direction for the surgeon during surgery, and the body axis direction (direction along the body axis) for the patient during surgery).

In addition, the case where the observation optical system includes a pair of left and right optical systems (optical systems that allow the user to perform binocular observation) will be mainly described below. However, an observation optical system of some other aspects may have an optical system for monocular observation, and it will be understood by those skilled in the art that the configuration described below may be incorporated into the aspects for monocular observation.

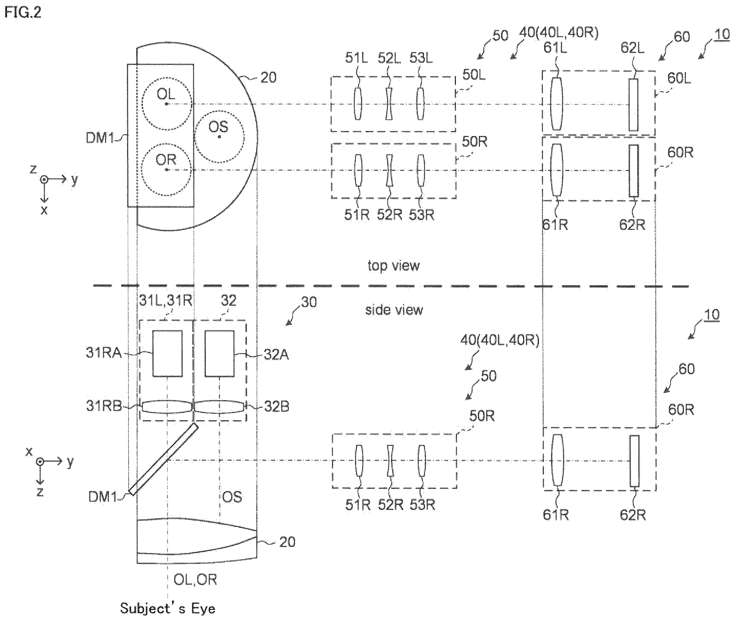

FIG. 2 shows an example of the configuration of the optical system of the surgical microscope 10. FIG. 2 illustrates a schematic top view of the optical system viewed from above (top view) and a schematic side view of the optical system viewed from the side (side view) in association with each other. In order to simplify the illustration, the illumination optical system 30 arranged above the objective lens 20 is omitted in the top view.

The surgical microscope 10 includes the objective lens 20, the dichroic mirror DM1, the illumination optical system 30, and the observation optical system 40. The observation optical system 40 includes the zoom expander 50, and the imaging camera 60. In some aspects, the illumination optical system 30 or the observation optical system 40 includes the dichroic mirror DM1.

The objective lens 20 is arranged to face the subject's eye. The objective lens 20 is arranged such that its optical axis is oriented along the z direction. The objective lens 20 may include two or more lenses.

The dichroic mirror DM1 couples the optical path of the illumination optical system 30 and the optical path of the observation optical system 40 with each other. The dichroic mirror DM1 is arranged between the illumination optical system 30 and the objective lens 20. The dichroic mirror DM1 transmits illumination light from the illumination optical system 30 and directs the illumination light to the subject's eye through the objective lens 20. Also, the dichroic mirror DM1 reflects return light from the subject's eye incident through the objective lens 20 and directs the return light to the imaging camera 60 of the observation optical system 40.

The dichroic mirror DM1 coaxially couples the optical path of the illumination optical system 30 and the optical path of the observation optical system 40 with each other. In other words, the optical axis of the illumination optical system 30 and the optical axis of the observation optical system 40 intersect at the dichroic mirror DM1. In the case where the illumination optical system 30 includes an illumination optical system for left eye (31L) and an illumination optical system for right eye (31R) and where the observation optical system 40 includes an observation optical system for left eye 40L and an observation optical system for right eye 40R, the dichroic mirror DM1 coaxially couples the optical path of the illumination optical system for left eye (the first illumination optical system 31L) and the optical path of the observation optical system for left eye 40L with each other, and coaxially couples the optical path of the illumination optical system for right eye (the first illumination optical system 31R) and the optical path of the observation optical system for right eye 40R with each other.

The illumination optical system 30 is an optical system for illuminating the subject's eye through the objective lens 20. The illumination optical system 30 may be configured to selectively illuminate the subject's eye with two or more pieces of illumination light having different color temperatures. The illumination optical system 30 projects illumination light having a designated color temperature onto the subject's eye under the control of a controller (the controller 200 described later).

The illumination optical system 30 includes the first illumination optical systems 31L and 31R and the second illumination optical system 32.

Each of the optical axis OL of the first illumination optical system 31L and the optical axis OR of the first illumination optical system 31R is arranged with the optical axis of the objective lens 20 in a substantially coaxial manner. Such arrangements enable a coaxial illumination mode and therefore make it possible to obtain a red reflex image (transillumination image) formed by utilizing diffuse reflection from eye fundus. The present aspect allows the red reflex image of the subject's eye to be observed with both eyes.

The second illumination optical system 32 is arranged in such a manner that its optical axis OS is eccentric (deviated, shifted) from the optical axis of the objective lens 20. The first illumination optical systems 31L and 31R and the second illumination optical system 32 are arranged such that the deviation of the optical axis OS with respect to the optical axis of the objective lens 20 is larger than the deviations of the optical axes OL and OR with respect to the optical axis of the objective lens 20. Such arrangements enable an illumination mode referred to as "angled illumination (oblique illumination)" and therefore enables binocular observation of the subject's eye while preventing ghosting caused by corneal reflection or the like. In addition, the arrangements enable detailed observation of unevenness and irregularities of sites and tissues of the subject's eye.

The first illumination optical system 31L includes the light source 31LA and the condenser lens 31LB. The light source 31LA outputs illumination light having a wavelength in the visible range (visible region) corresponding to color temperature of 3000 K (kelvins), for example. The illumination light emitted from the light source 31LA passes through the condenser lens 31LB, passes through the dichroic mirror DM1, passes through the objective lens 20, and then is incident on the subject's eye.

The first illumination optical system 31R includes the light source 31RA and the condenser lens 31RB. The light source 31RA also outputs illumination light having a wavelength in the visible range corresponding to color temperature of 3000 K, for example. The illumination light emitted from the light source 31RA passes through the condenser lens 31RB, passes through the dichroic mirror DM1, passes through the objective lens 20, and then is incident on the subject's eye.

The second illumination optical system 32 includes the light source 32A and the condenser lens 32B. The light source 32A outputs illumination light having a wavelength in the visible range corresponding to a color temperature within the range of 4000 K to 6000 K, for example. The illumination light emitted from the light source 32A passes through the condenser lens 32B, passes through the objective lens 20 without passing through the dichroic mirror DM1, and then is incident on the subject's eye.

In the present aspect example, the color temperature of the illumination light from the first illumination optical systems 31L and 31R is lower than the color temperature of the illumination light from the second illumination optical system 32. Such a configuration makes it possible to observe the subject's eye in warm colors using the first illumination optical systems 31L and 31R, and therefore enables detailed observation of the structure and morphology of the subject's eye.

In some aspects, each of the optical axes OL and OR is movable relative to the optical axis of the objective lens 20. The direction of the relative movement is a direction that intersects the optical axis of the objective lens 20, and the relative movement is represented by a displacement vector in which at least one of the x component and the y component is not zero. In some aspects, the optical axes OL and OR may be mutually independently movable. On the other hand, in some aspects, the optical axes OL and OR may be integrally movable. For example, the surgical microscope 10 includes a movement mechanism (31d) configured to move the first illumination optical systems 31L and 31R mutually independently or integrally, and therefore the movement mechanism moves the first illumination optical systems 31L and 31R mutually independently or integrally in a direction intersecting the optical axis of the objective lens 20. Such a configuration makes it possible to conduct adjustment of the appearance condition (appearance state) of the subject's eye. In some aspects, the movement mechanism operates under the control of a controller (the controller 200 described later).

In some aspects, the optical axis OS is movable relative to the optical axis of the objective lens 20. The direction of the relative movement is a direction that intersects the optical axis of the objective lens 20, and the relative movement is represented by a displacement vector in which at least one of the x component and the y component is not zero. For example, the surgical microscope 10 includes a movement mechanism (32d) configured to move the second illumination optical system 32, and therefore the movement mechanism moves the second illumination optical system 32 in a direction that intersects the optical axis of the objective lens 20. With such a configuration, it becomes possible to conduct adjustment of the appearance condition (appearance state) of unevenness and irregularities of sites and tissues of the subject's eye. In some aspects, the movement mechanism operates under the control of a controller (the controller 200 described later).

As described above, the present aspect is configured such that the illumination optical system 30 is arranged at the position directly above the objective lens 20 (the position in the transmission direction of the dichroic mirror DM1) and the observation optical system 40 is arranged at the position in the reflection direction of the dichroic mirror DM1. For example, the observation optical system 40 may be arranged in such a manner that the angle formed by the optical axis of the observation optical system 40 and the plane perpendicular to the optical axis of the objective lens 20 (the xy plane) belongs to the range between -20 degrees and +20 degrees.

According to the configuration of the present aspect, the observation optical system 40, which typically has a longer optical path length than the illumination optical system 30, is arranged substantially parallel to the xy plane. Hence, the observation optical system 40 of the present aspect does not interfere with the surgeon's field of view while conventional surgical microscopes, whose observation optical system is oriented along the vertical direction in front of the surgeon's eyes, do. Therefore, the surgeon is capable of easily seeing the screen of the display device 3 arranged in front of the surgeon. In other words, the visibility of displayed information (images and videos of the subject's eye, and other various kinds of reference information) during surgery etc. is improved. In addition, since the housing is not placed in front of the surgeon's eyes, it does not give a sense of oppression to the surgeon, thereby reducing the burden on the surgeon.

The observation optical system 40 is an optical system for observation of an image formed based on return light of the illumination light incident from the subject's eye through the objective lens 20. In the present aspect, the observation optical system 40 guides the image to an image sensor of the imaging camera 60.

As described above, the observation optical system 40 includes the observation optical system for left eye 40L and the observation optical system for right eye 40R. The configuration of the observation optical system for left eye 40L and the configuration of the observation optical system for right eye 40R are the same as or similar to one another. In some aspects, the observation optical system for left eye 40L and the observation optical system for right eye 40R may be configured in such a manner that their optical arrangements can be changed independently of each other.

The zoom expander 50 is also referred to as a beam expander, a variable beam expander, or the like. The zoom expander 50 includes the zoom expander for left eye 50L and the zoom expander for right eye 50R. The configuration of the zoom expander for left eye 50L and the configuration of the zoom expander for right eye 50R are the same as or similar to each other. In some aspects, the zoom expander for left eye 50L and the zoom expander for right eye 50R may be configured in such a manner that their optical arrangements can be changed independently of each other.

The zoom expander for left eye 50L includes the plurality of zoom lenses 51L, 52L, and 53L. At least one of the zoom lenses 51L, 52L, and 53L is movable in the direction along the optical axis with a variable magnification mechanism (not shown in the drawings).

Similarly, the zoom expander for right eye 50R includes the plurality of zoom lenses 51R, 52R, and 53R, and at least one of the zoom lenses 51R, 52R, and 53R is movable in the direction along the optical axis with a variable magnification mechanism (not shown in the drawings).

The variable magnification mechanism(s) may be configured to move a zoom lens of the zoom expander for left eye 50L and a zoom lens of the zoom expander for right eye 50R mutually independently or integrally in the directions along the optical axes. As a result of this, the magnification ratio for photographing the subject's eye is changed. In some aspects, the variable magnification mechanism(s) operates under the control of a controller (the controller 200 described later).

The imaging camera 60 is a device that photographs an image formed by the observation optical system 40 and generates digital image data. The imaging camera 60 is typically a digital camera (digital video camera). The imaging camera 60 includes the imaging camera for left eye 60L and the imaging camera for right eye 60R. The configuration of the imaging camera for left eye 60L and the configuration of the imaging camera for right eye 60R are the same as or similar to one another. In some aspects, the imaging camera for left eye 60L and the imaging camera for right eye 60R may be configured such that their optical arrangements can be changed independently of each other.

The imaging camera for left eye 60L includes the imaging lens 61L and the image sensor 62L. The imaging lens 61L forms an image based on the return light that has passed through the zoom expander for left eye 50L, on the imaging surface (light receiving surface) of the image sensor 62L. The image sensor 62L is an area sensor, and may typically be a charge-coupled device (CCD) image sensor or a complementary metal oxide semiconductor (CMOS) image sensor. The image sensor 62L operates under the control of a controller (the controller 200 described later).

The imaging camera for right eye 60R includes the imaging lens 61R and the image sensor 62R. The imaging lens 61R forms an image based on the return light that has passed through the zoom expander for right eye 50R, on the imaging surface (light receiving surface) of the image sensor 62R. The image sensor 62R is an area sensor, and may typically be a CCD image sensor or a CMOS image sensor. The image sensor 62R operates under the control of a controller (the controller 200 described later).

### < Processing system >

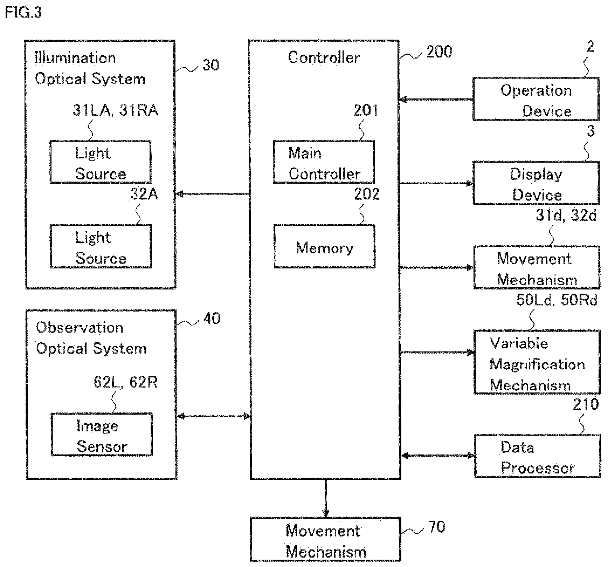

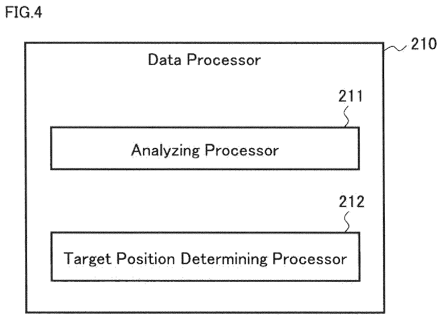

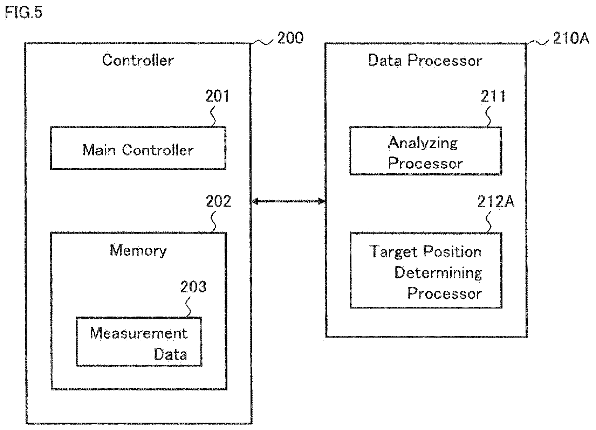

The processing system of the ophthalmic observation apparatus 1 will be described. Examples of a basic configuration of the processing system are shown in FIG. 3 and FIG. 4, and some examples of specific configurations are shown in FIG. 5, FIG.6, and FIG.7. Any two or more of the various kinds of configuration examples described below may be combined at least in part. Note that the configuration of the processing system is not limited to the examples described below.

The controller 200 executes a control of each part of the ophthalmic observation apparatus 1. The controller 200 includes the main controller 201 and the memory 202. The main controller 201 includes a processor and executes a control of each part of the ophthalmic observation apparatus 1. For example, the processor may load and run a program stored in the memory 202 or another storage device, thereby implementing a function according to the present aspect. In addition, the processor may use (e.g., referring, processing, calculating, etc.) data and/or information stored in the memory 202 or another storage device in order to implement a function according to the present aspect.

The main controller 201 may control the light sources 31LA, 31RA, and 32A of the illumination optical system 30, the image sensors 62L and 62R of the observation optical system 40, the movement mechanisms 31d and 32d, the variable magnification mechanisms 50Ld and 50Rd, the operation device 2, the display device 3, and other component parts.

Controls of the light source 31LA include turning on and off the light source, adjusting the light amount, adjusting the diaphragm (aperture), and so forth. Controls of the light source 31RA include turning on and off the light source, adjusting the light amount, adjusting the diaphragm (aperture), and so forth. The main controller 201 may perform mutually exclusive controls of the light sources 31LA and 31RA. Controls of the light source 32A include turning on and off the light source, adjusting the light amount, adjusting the diaphragm (aperture), and so forth.

In the case where the illumination optical system 30 includes a light source whose color temperature can be varied, the main controller 201 may change the color temperature of emitted illumination light by controlling such a light source.

Controls of the image sensor 62L include exposure adjustment, gain adjustment, photographing rate adjustment, and so forth. Controls of the image sensor 62R include exposure adjustment, gain adjustment, photographing rate adjustment, and so forth. Further, the main controller 201 may control the image sensors 62L and 62R in such a manner that the photographing timings of the image sensors 62L and 62R match each other, or in such a manner that the difference between the photographing timings of the image sensors 62L and 62R lies within a predetermined time. In addition, the main controller 201 may perform a control of loading digital data obtained by the image sensors 62L and 62R.

The movement mechanism 31d moves the light sources 31LA and 31RA mutually independently or integrally in a direction that intersects the optical axis of the objective lens 20. By controlling the movement mechanism 31d, the main controller 201 moves the optical axes OL and OR mutually independently or integrally with respect to the optical axis of the objective lens 20.

The movement mechanism 32d moves the light source 32A in a direction that intersects the optical axis of the objective lens 20. By controlling the movement mechanism 32d, the main controller 201 moves the optical axis OS with respect to the optical axis of the objective lens 20.

The movement mechanism 70 moves the surgical microscope 10. For example, the movement mechanism 70 is configured to integrally move at least part of the illumination optical system 30 and the observation optical system 40. This configuration makes it possible to change the relative positions of the at least part of the illumination optical system 30 and the observation optical system 40 with respect to the subject's eye while maintaining the relative positional relationship between at least part of the illumination optical system 30 and the observation optical system 40. In some aspects, the movement mechanism 70 is configured to integrally move the first illumination optical systems 31L and 31R and the observation optical system 40. With this, the relative positions of the first illumination optical systems 31L and 31R with respect to the subject's eye and the relative position of the observation optical system 40 with respect to the subject's eye can be changed while maintaining the state (condition) of coaxial illumination. In some aspects, the movement mechanism 70 is configured to integrally move the second illumination optical system 32 and the observation optical system 40. With this, the relative positions of the second illumination optical system 32 and the observation optical system 40 with respect to the subject's eye can be changed while maintaining the illumination angle for oblique illumination. In some aspects, the movement mechanism 70 is configured to integrally move the first illumination optical systems 31L and 31R, the second illumination optical system 32, and the observation optical system 40. This makes it possible to change the relative positions of the illumination optical system 30 and the observation optical system 40 with respect to the subject's eye while maintaining both the state (condition) of coaxial illumination and the illumination angle for oblique illumination. The movement mechanism 70 operates under a control of the controller 200.

In some aspects, the main controller 201 may be configured to control at least two of the movement mechanisms 31d, 32d, and 70 in an interlocking manner.

The variable magnification mechanism 50Ld moves at least one of the plurality of zoom lenses 51L to 53L of the zoom expander for left eye 50L in the optical axis direction (direction along the optical axis). The main controller 201 changes the magnification ratio of the observation optical system for left eye 40L by controlling the variable magnification mechanism 50Ld.

Similarly, the variable magnification mechanism 50Rd moves at least one of the plurality of zoom lenses 51R to 53R of the zoom expander for right eye 50R in the optical axis direction (direction along the optical axis). The main controller 201 changes the magnification ratio of the observation optical system for right eye 40R by controlling the variable magnification mechanism 50Rd.

Controls for the operation device 2 include an operation permission control, an operation prohibition control, an operation signal transmission control and/or an operation signal reception control from the operation device 2, and other controls. The main controller 201 receives an operation signal generated by the operation device 2 and executes a control corresponding to the operation signal received.

Controls for the display device 3 include an information display control and other controls. As a display controller, the main controller 201 displays various kinds of information on the display device 3. For example, the main controller 201 displays an image based on digital image data generated by the image sensors 62L and 62R on the display device 3. Typically, the main controller 201 may display a moving image (video, movie) based on digital image data (video signal) generated by the image sensors 62L and 62R on the display device 3. Further, the main controller 201 may display a still image (frame) included in the moving image on the display device 3. In addition, the main controller 201 may display an image (a moving image, a still image, etc.) obtained by processing the digital image data generated by the image sensors 62L and 62R on the display device 3. Furthermore, the main controller 201 may display, on the display device 3, any information generated by the ophthalmic observation apparatus 1, any information acquired from the outside by the ophthalmic observation apparatus 1, and other types of information.

Further, the main controller 201 may create an image for left eye from the digital image data generated by the image sensor 62L and create an image for right eye from the digital image data generated by the image sensor 62R, and then display the created image for left eye and the created image for right eye on the display device 3 in such a manner as to enable stereoscopic vision. For example, the main controller 201 may create a pair of left and right parallax images from the image for left eye and the image for right eye, and display the pair of parallax images on the display device 3. With this, the user (e.g., surgeon) can recognize the pair of parallax images as a stereoscopic image by using a known stereoscopic method or technique. The stereoscopic method applicable to the present aspect may be freely selected, and for example, may be any of the following methods: a stereoscopic method for naked eyes; a stereoscopic method using an auxiliary device (polarized glasses, etc.); a stereoscopic method by applying image processing (image synthesis, image composition, rendering, etc.) to an image for left eye and an image for right eye; a stereoscopic method by displaying a pair of parallax images simultaneously; a stereoscopic method by alternately displaying a pair of parallax images; and a stereoscopic method of a combination of two or more of the above methods.

The data processor 210 executes various kinds of data processes. Some examples of processing that may be executed by the data processor 210 will be described below. The data processor 210 (each element thereof) includes a processor that operates on the basis of predetermined software (program), and is implemented by the cooperation of hardware and software.

Some examples of processing that may be executed by the data processor 210 will be described together with related elements. FIG. 4 shows an example of a basic configuration of the data processor 210. The data processor 210 includes the analyzing processor 211 and the target position determining processor 212.

The surgical microscope 10 is configured to generate a moving image (live image) by photographing the subject's eye. The controller 200 is configured to capture a moving image frame (still image) and input the captured moving image frame into the analyzing processor 211. In some examples, the controller 200 captures a frame from a moving image in response to a manual or automatic trigger (upon receiving a manual or automatic trigger).

The analyzing processor 211 is configured to analyze a frame captured from the moving image to detect an image of a predetermined site of the subject's eye, that is, to detect an image region corresponding to a predetermined site of the subject's eye. The image region to be detected from the frame by the analyzing processor 211 may be, for example, an image region identified as an image of the predetermined site of the subject's eye, or an image region obtained by processing the image of the predetermined site of the subject's eye (and its vicinity). Examples of the image region obtained by processing the image of the predetermined site of the subject's eye (and its vicinity) include an approximate figure such as an approximate ellipse or an approximate circle.

The predetermined site of the subject's eye detected by the analyzing processor 211 is the cornea (its entirety, or a feature site such as the corneal ring, the corneal edge, the corneal center, or the corneal apex).

The analyzing processor 211 may be configured to detect an image of a predetermined site of the subject's eye from a still image using a freely selected region extraction method or technique. In some examples of detecting an image of a site characterized by its brightness (an image of a site that has a distinctive feature in brightness), the analyzing processor 211 may be configured to perform detection of an image of this site of the subject's eye from a still image using brightness thresholding such as binarization. In the case of detecting an image of a site characterized by its shape (an image of a site that has a distinctive feature in shape), the analyzing processor 211 may be configured to perform detection of an image of this site of the subject's eye from a still image using shape analysis processing such as pattern matching. In the case of detecting an image of a site characterized by its color tone (an image of a site that has a distinctive feature in color tone), the analyzing processor 211 may be configured to perform detection of an image of this site of the subject's eye from a still image using color analysis processing such as feature color extraction. In some examples, the analyzing processor 211 may be configured to detect an image of a predetermined site of the subject's eye by applying segmentation to a still image to identify an image of this site of the subject's eye. Typically, segmentation is image processing for identifying a partial region (subregion) in a given image. Segmentation may include any known image processing technique, and some examples of which may include image processing such as edge detection and/or machine learning (e.g., deep learning) based segmentation.

The image of the predetermined site of the subject's eye (information that represents the position of the image, the area of the image, etc.) detected by the analyzing processor 211 is input into the target position determining processor 212. The target position determining processor 212 is configured to determine a target position for a predetermined treatment (a predetermined medical treatment, a predetermined procedure, a predetermined medical procedure) based at least on the image of the predetermined site detected by the analyzing processor 211.

The predetermined treatment, which is not according to the claimed invention, may be any one or more of the following items in cataract surgery: incision creation (corneal incision, sclerocorneal incision), ophthalmic viscosurgical device (OVD) injection, crystalline lens anterior capsulotomy (CCC), crystalline lens phacoemulsification and aspiration, IOL insertion, ophthalmic viscosurgical device removal, and incision closure. In some examples, the predetermined treatment may be any of the following treatment items: any treatment conducted in refractive surgery, any treatment conducted in minimally invasive glaucoma surgery (MIGS), any treatment conducted in vitreoretinal surgery, and any treatment conducted in other ophthalmic surgery.

Several examples of the target position determination processing will be described. In the first example, which is not part of the invention, preliminary measurement (preparatory measurement) is performed on the subject's eye. Measurement data obtained from the subject's eye by the preliminary measurement is stored in the memory 202 of the ophthalmic observation apparatus 1 or stored in a storage device accessible by the ophthalmic observation apparatus 1. The first example can be implemented by means of the configuration example shown in FIG. 5. The data processor 210A is an example of the data processor 210 of FIG. 4, and the target position determining processor 212A is an example of the target position determining processor 212 of FIG. 4.

In some aspect examples where the predetermined treatment is ocular incision (e.g., corneal incision, sclerocorneal incision, conjunctival incision, scleral incision, etc.), the preliminary measurement may be any of the following measurement items, for example: aberration measurement of the subject's eye by means of a wavefront sensor, corneal topography (corneal shape measurement) of the subject's eye by means of a corneal topographer, and corneal topography of the subject's eye by means of an anterior eye segment OCT apparatus. Examples of the wavefront sensor and the corneal topographer are described, for example, in International Publication No. WO 2003/022138. Anterior eye segment OCT is described, for example, in International Publication No. WO 2017/154348.

The measurement data 203 shown in FIG. 5 is an example of data obtained from the subject's eye by the preliminary measurement. The measurement data 203 may include, for example, aberration data (corneal aberration data) of the subject's eye obtained using the wavefront sensor, corneal shape data of the subject's eye obtained using the corneal topographer, and corneal shape data of the subject's eye obtained using the anterior eye segment OCT apparatus.

The target position determining processor 212A of the first example is configured to determine a target position for the predetermined treatment based at least on the image of the predetermined site of the subject's eye detected by the analyzing processor 211 and the measurement data 203. If the predetermined treatment is an ocular incision (e.g., corneal incision, sclerocorneal incision, etc.), the target position determining processor 212A may determine a position where corneal astigmatism that can result from the incision is reduced the most.

For example, based on the measurement data 203 (e.g., corneal shape data, corneal aberration data, etc.), the target position determining processor 212A can determine a position where the incision has little impact (influence, effect) on corneal astigmatism. Typically, an incision is made at a position of the steepest meridian (first principal meridian, principal meridian, major meridian) of the cornea in order to correct corneal astigmatism before the incision by incision-induced astigmatism (steepest meridian incision treatment). In this case, the measurement data 203 may include data that represents the steepest meridian position of the cornea of the subject's eye, that is, data that represents an angle direction of the steepest meridian. In a wider sense, the measurement data 203 may include data that represents an approximate target position (rough target position) in the subject's eye used to determine a specific target position (accurate target position).

For example, the target position determining processor 212A may be configured to identify a position corresponding to the direction of the steepest meridian in the corneal ring (or, a position corresponding to the direction of the steepest meridian on a closed curve apart from the corneal ring in an outward direction by a predetermined distance) of the subject's eye, based on steepest meridian data included in the measurement data 203 or steepest meridian data derived from the measurement data 203 and on an image of the corneal ring detected by the analyzing processor 211. Then, the target position determining processor 212A can set the identified position as a target incision position of the subject's eye.

In a wider sense, the target position determining processor 212A may be configured to identify a position corresponding to an approximate target position in the predetermined site (or, a position corresponding to an approximate target position in a location, area, or site that satisfies a predetermined condition with respect to the predetermined site) of the subject's eye, based on an approximate target position included in the measurement data 203 or an approximate target position determined from the measurement data 203 and on an image of the predetermined site detected by the analyzing processor 211. Then, the target position determining processor 212A can set the identified position as a target application position of the predetermined treatment for the subject's eye.

The number of target positions determined by the target position determining processor 212A may be freely determined. In the case where the predetermined treatment is an ocular incision, the target position determining processor 212A may be configured to determine, for example, a position of a main port (main wound, main incision) and one or more positions of one or more side ports (one or more side wounds, one or more side incisions) may be determined. The number and/or positions of target positions may be set based on various kinds of conditions such as the type of the predetermined treatment, the kind or degree of disease, the kind of instrument used, and doctor's preference.

In the second example of the target position determination processing, which is not part of the invention, measurement of the subject's eye is performed by the ophthalmic observation apparatus 1 (or by a system including the ophthalmic observation apparatus 1). In other words, the second example does not require preliminary measurement as in the first example to be performed. Therefore, the second example can be employed even in the case where measurement data is not prepared in advance.

Note that the second example may be operated, optionally, to refer to measurement data obtained by preliminary measurement. For example, some aspect examples may be configured to process both the first measurement data obtained by the preliminary measurement and the second measurement data obtained by using the ophthalmic observation apparatus 1 to generate the third measurement data, and then to perform target position determination processing on the basis of the third measurement data. The processing for generating the third measurement data from the first and second measurement data may include freely selected or designed statistical processing.

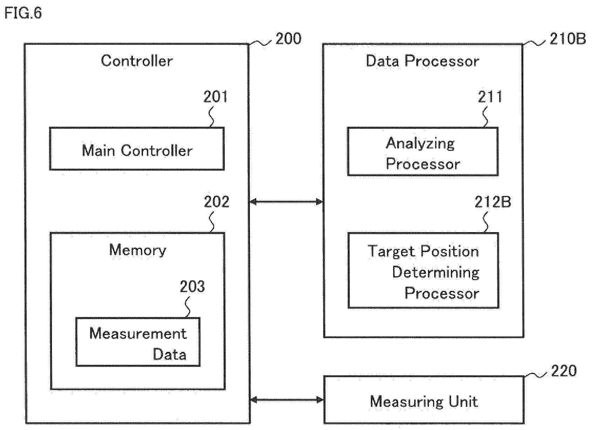

The measurement data obtained from the subject's eye by means of the ophthalmic observation apparatus 1 is stored in the memory 202 of the ophthalmic observation apparatus 1, or stored in a storage device accessible by the ophthalmic observation apparatus 1. The configuration example shown by FIG. 6 may be applied to the second example. The data processor 210B is an example of the data processor 210 of FIG. 4, and the target position determining processor 212B is an example of the target position determining processor 212 of FIG. 4.

The ophthalmic observation apparatus 1 shown by FIG. 6 includes the measuring unit 220. The measuring unit 220 has an ophthalmic measuring function for obtaining measurement data of the subject's eye. Similar to the ophthalmic measurement apparatus to which the preliminary measurement of the first example is also applied, the measuring unit 220 includes any of a wavefront sensor, a corneal topographer, and an anterior eye segment OCT apparatus in the case where the predetermined treatment is an ocular incision.

The measurement data 203 shown in FIG. 6 is an example of data acquired from the subject's eye by means of the measuring unit 220. The measurement data 203 may include, for example, any of the following kinds of data: aberration data (corneal aberration data) of the subject's eye acquired by means of the wavefront sensor; corneal shape data of the subject's eye acquired by means of the corneal topographer; and corneal shape data of the subject's eye acquired by means of the anterior eye segment OCT apparatus.

The target position determining processor 212B of the second example is configured to determine the target position for the predetermined treatment based at least on the image of the predetermined site of the subject's eye detected by the analyzing processor 211 and the measurement data 203 acquired by the measuring unit 220.

Similar to the target position determining processor 212A of the first example, if the predetermined treatment is an ocular incision, the target position determining processor 212B may be configured to determine a position where corneal astigmatism that can result from the incision is minimized. The attributes of the target position and the content of the target position determination processing in the second example may be the same as or similar to those in the first example.

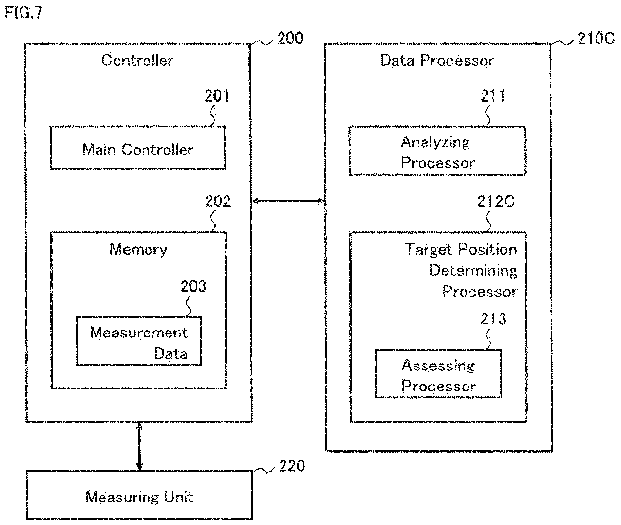

As in the case of considering corneal astigmatism that can be caused by an ocular incision, in the case where the target position is determined in consideration of the influence of the predetermined treatment on the subject's eye, the configuration example shown in FIG. 7 may be employed. The data processor 210C is an example of the data processor 210B of FIG. 6, and the target position determining processor 212C is an example of the target position determining processor 212B of FIG. 6.

The target position determining processor 212C includes the assessing processor 213. The assessing processor 213 is configured to assess influence of the predetermined treatment on the subject's eye based on the measurement data of the subject's eye acquired by the measuring unit 220. For example, the assessing processor 213 assesses influence of an ocular incision (e.g., corneal incision, sclerocorneal incision, etc.) on corneal astigmatism of the subject's eye. In some aspects, the assessment processing may include, for example, any of the following processes: a process of determining a direction of the steepest meridian from corneal shape data or corneal aberration data; a process of determining the refractive power in the direction of the steepest meridian; a process of determining a direction of a flat meridian (flatter meridian, flattest meridian, second principal meridian, minor meridian); a process of determining the refractive power in the direction of the flat meridian; and a process of obtaining a distribution of refractive powers.

Furthermore, the assessment processing includes a simulation of the state (condition) of the cornea after an ocular incision based at least on the state of the cornea before the ocular incision. The state of the cornea may be any one or more of corneal shape data, corneal aberration data, the direction of the steepest meridian, the refractive power in the direction of the steepest meridian, the direction of the flat meridian, the refractive power in the direction of the flat meridian, the refractive power distribution, and like information. A simulation of some examples may include a rule-based processing performed according to a predetermined simulation program. Also, a simulation of some examples may include processing performed by means of a machine learning system (e.g., neural network, inference model) constructed by supervised learning with training data that includes states of corneas before ocular incisions, attributes of ocular incisions (e.g., incision positions, incision shapes, incision sizes), and states of corneas after ocular incisions. Simulations that can be performed by the assessing processor 213 are not limited to these examples. The assessing processor 213 of some examples may be configured to execute a simulation by means of other methods or techniques such as unsupervised learning, or may be configured to execute a simulation by means of an at-least-in-part combination of two or more methods or techniques.

The target position determining processor 212C of FIG. 7 is configured to determine the target position for the predetermined treatment for the subject's eye based at least on the image of the predetermined site detected by the analyzing processor 211 and a result acquired by the assessing processor 213. For example, the target position determining processor 212C may determine, as the target position for the predetermined treatment for the subject's eye, a position where corneal astigmatism that can result from the ocular incision is expected to be the least amount.

In the third example of the target position determination processing, the target position for the predetermined treatment for the subject's eye is determined without referring to measurement data of the subject's eye as described by the first and second examples. The configuration example of FIG. 4 (or any of the configuration examples of FIG. 5 to FIG. 7) may be employed to the third example. The target position determining processor 212 of the third example is configured to determine, as the target position for the predetermined treatment for the subject's eye, a position away from the image of the predetermined site of the subject's eye detected by the analyzing processor 211 by a predetermined distance. The predetermined distance is defined, for example, as a real distance (e.g., millimeters, micrometers, or the like) or as an image distance (e.g., number of pixels, or the like).

For example, in an anterior capsulotomy (CCC) for cataract surgery, the analyzing processor 211 may find a feature of the anterior segment of the subject's eye. Here, the feature of the anterior eye segment of the subject's eye may include, for example, any of the following kinds of information: an image of the corneal ring; a size of the image of the corneal ring; an image of the pupil edge; and a size of the image of the pupil edge. Based on the feature of the anterior eye segment obtained by the analyzing processor 211, the target position determining processor 212 may determine a figure that has a predetermined shape and a predetermined size. This figure is, for example, a circle with a diameter of 6 millimeters. This circle may be any of the following circles, for example: a circle with a radius of 3 millimeters centered at the center of an approximate circle (or an approximate ellipse) to the corneal ring; a circle with a radius of 3 millimeters centered at the center of an approximate circle (or an approximate ellipse) to the pupil edge; a circle with a diameter of 6 millimeters that is obtained by enlarging (or reducing) an approximate circle to the corneal ring in an isotropic manner; a circle of a diameter of 6 millimeters which is obtained by enlarging (reducing) an approximate circle to the pupil edge in an isotropic manner. It should be noted that the shape, size, or other parameters of the figure may be changed according to user's preference.

Further, the analyzing processor 211 may be configured to detect an image of the corneal ring of the subject's eye in vitreoretinal surgery. The target position determining processor 212 may be configured to set, as a target position for inserting a surgical instrument, a position away from the detected image of the corneal ring in an outward direction by a predetermined distance (e.g., 3 to 4 millimeters). Note that the shape, size, or other parameters of the figure may be changed according to user's preference.

An example of target position determination processing (including distance calculation processing) applicable to the third example (or other examples) will be described. The following is a description of the case where the predetermined site of the subject's eye detected by the analyzing processor 211 is a corneal ring (iris outer edge) is considered. The same or similar processing can also be applied to the cases where other sites (e.g., the pupil edge) are considered.

As preliminary processes (preparatory processes), the following processes may be performed prior to surgery: performing photography of the anterior eye segment of the subject's eye; recording the distance between the subject's eye and the photographing apparatus (working distance (WD)) at the time of the photography; detecting the corneal ring from the photographed image obtained in the photography; measuring the size (e.g., diameter) of the corneal ring detected; and recording the value obtained by the size measurement. Note that the method or technique applied to the preliminary operation of obtaining corneal ring size may be freely selected or determined. For example, corneal ring size may be measured from an OCT image of the anterior eye segment (e.g., cross sectional image or three dimensional image), or may be measured directly without using an image.

Under the above preparations, the following processes may be performed during the surgery: acquiring an observation image by performing photography of the subject' eye by means of the ophthalmic observation apparatus 1 (the surgical microscope 10): recording a working distance at the time of the photography (by means of the controller 200); detecting the corneal ring from the observation image obtained in the photography (by means of the analyzing processor 211); measuring the size of the corneal ring detected (by means of the data processor 210); and recording a value obtained by the corneal ring size measurement (by means of the controller 200).

Furthermore, based on the relationship between the working distance at the time of the preoperative photography and the working distance at the time of the intraoperative photography (e.g., the ratio of the two working distances), the target position determining processor 212 finds the relationship between the scale (e.g., actual distance per pixel) of the photographed image obtained prior to the surgery and the scale of the observation image obtained during the surgery (e.g., the ratio of the scales of the two images). Furthermore, based on the relationship between the scale of the preoperative image and the scale of the intraoperative image thus determined, the target position determining processor 212 identifies the position (location, area, site) that is a predetermined distance (e.g., 3 to 4 millimeters) outward from the image of the corneal ring detected in the intraoperative image. In the present example, the controller 200 (the main controller 201) may display an observation image (live image) of the subject's eye on the display device 3, and also display target position information that represents the identified position (target position) on the observation image. The target position information may be in any form. Examples of the form of the target position information may be a circular indicator, elliptic indicator, or arc-shaped indicator.

Some modification examples will be described. In the preoperative photography, photography may be performed with a ruler applied to the subject's eye (anterior eye segment), and the actual distance may be measured based on the ruler depicted in the photographed image obtained. Likewise, in the intraoperative photography, photography may be performed with a ruler applied to the subject's eye (anterior eye segment), and the actual distance may be measured based on the ruler depicted in the photographed image obtained. While the distance from the corneal ring to the target position is 3 to 4 millimeters in the above example, the distance may be freely determined. In some examples, the distance from the corneal ring to the target position may be determined based on any of surgeon's preference, corneal shape (e.g., corneal curvature or corneal curvature distribution), and other factors.

In the case where any of the configuration examples of FIG. 4 to FIG. 7 or another configuration example is applied, the controller 200 receives a live image of the subject's eye from the surgical microscope 10 and displays the live image on the display device 3. At the same time, the controller 200 displays, on the display device 3, the target position information that represents the target position determined by the analyzing processor 211 and the target position determining processor 212.

According to the ophthalmic observation apparatus 1 configured in this way, the target position for the predetermined treatment to be applied to the subject's eye can be provided to the user together with a live image, so that the user can see and understand in real time the position to which the predetermined treatment should be applied. This makes it possible to facilitate surgery (or treatment etc.), shorten the time required for surgery (or treatment etc.), reduce or prevent mistakes or errors in treatment, reduce burdens on the patient, and so forth.

The ophthalmic observation apparatus 1 may be configured to execute real time updates of the target position information displayed together with the live image generated by the surgical microscope 10. In order to implement this real time update function, the analyzing processor 211 may be configured to sequentially detect images of the predetermined site of the subject's eye from frames (still images) sequentially generated as a moving image by the surgical microscope 10, in parallel with generation of the moving image performed by the surgical microscope 10. In parallel with both the generation of the moving image and the detection of the images, the target position determining processor 212 sequentially determines the target position for the predetermined treatment based at least on the images of the predetermined site sequentially detected by the analyzing processor 211. In parallel with the generation of the moving image, the detection of the images, and the target position determination, the controller 200 displays a live image (sequential updating of frames) and perform updates of the target position information (sequential switching of the target position information) based on the target positions sequentially determined by the target position determining processor 212. According to such a configuration, the user can become aware, in real time, of a change in the target position for the predetermined treatment while observing a live image of the subject's eye. Note that the change in the target position is caused by factors such as the movement of the subject's eye.

When two or more treatments are performed sequentially or in parallel, the ophthalmic observation apparatus 1 can perform different processing contents for individual treatments. For example, the following treatments are performed in cataract surgery: creation of an incision (corneal incision, sclerocorneal incision), injection of an ophthalmic viscosurgical device, anterior capsulotomy (CCC), phacoemulsification and aspiration, insertion of an IOL, removal of the ophthalmic viscosurgical device, and closure of the incision.

Typically, in the case where the first to N-th treatments are applied to the subject's eye, the analyzing processor 211 may analyze the n-th still image generated by the surgical microscope 10 prior to the n-th treatment, thereby detecting an image of the n-th site of the subject's eye.

Here, N is an integer equal to or greater than 2, and n is any integer belonging to the range between 1 and N. For any two integers n₁ and n₂ both belonging to the range between 1 and N (n₁ and n₂ are mutually different integers), the n₁-th treatment and the n₂-th treatment may be either the same kind of treatment or different kinds of treatments, and the n₁-th site and the n₂-th site may be either the same site or different sites.

Furthermore, the target position determining processor 212 may determine the n-th target position, which is the target position for the n-th treatment, based at least on an image of the n-th site detected from the n-th still image.

In addition, the controller 200 may display, for the n-th treatment, the live image acquired by the surgical microscope 10 and the n-th target position information that represents the n-th target position (n-th display control).

In the case where two or more treatments are performed sequentially (one after another), the ophthalmic observation apparatus 1 may be configured to execute automatic detection of transitions of treatments (shifting of treatments, switching of treatments). For example, the data processor 210 is configured to detect the transition from the first treatment to the second treatment (by means of a treatment transition detecting processor). It should be noted that the ophthalmic observation apparatus 1 may be configured to detect treatment transitions in response to an instruction issued by the user. The instruction may be input, for example, by a manual operation, voice, or the like.

Many surgeries and treatments are conducted according to predetermined procedures. For example, cataract surgery is conducted according to a series of procedures (a series of treatments) including incision creation, ophthalmic viscosurgical device injection, anterior capsulotomy, phacoemulsification and aspiration, IOL insertion, ophthalmic viscosurgical device removal, and incision closure. Taking this fact into account, as an operation mode corresponding to a specific surgery (or, a specific treatment or a specific therapy), processing contents (e.g., image detection, target position determination, target position information display, etc.) for individual procedures of the surgery can be determined and set in advance.

By referring to such preset information, the ophthalmic observation apparatus 1 is capable of automatically detecting an event that one procedure has completed and the next procedure (new procedure, new treatment) has been commenced, and further performing an operation mode associated in advance with the new procedure.

The automatic detection of procedure transitions (treatment transitions) may be implemented, for example, by analyzing a live image to detect the state of a specific site of the subject's eye and/or by analyzing a live image to detect an image of an instrument and/or an image of an intraocular implantation device.

For example, automatic detection of a transition to an anterior capsulotomy (CCC) procedure in cataract surgery **not** according to the claimed invention may be implemented by a combination of detection of a state in which a corneal incision has already been formed and detection of a state in which a treatment on the anterior lens capsule has not yet been performed. In some alternative examples, automatic detection of a transition to an anterior capsulotomy (CCC) procedure may be implemented by a combination of detection of a state in which a corneal incision has already been formed and detection of an image of an instrument for CCC.

Note that procedures or treatments to be automatically detected are not limited to anterior capsulotomy (CCC). Procedures or treatments to be automatically detected may be other procedures or treatments in cataract surgery, any procedures or treatments in any other kinds of surgery, or any kinds of procedures or treatments in any kinds of medical practices (treatments, therapies, diagnosis, etc.). Specific methods or techniques applied to automatic detections of transitions to procedures (treatments) other than anterior capsulotomy (CCC) may be performed in the same manner as the above example for anterior capsulotomy (CCC) and are not according to the claimed invention.

### < Operation and usage mode >

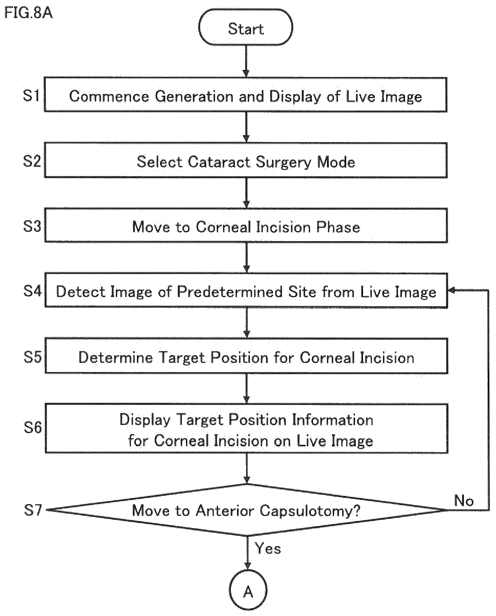

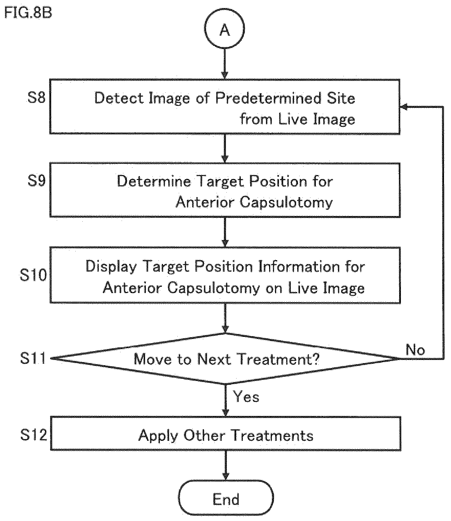

The operation and the usage mode of the ophthalmic observation apparatus 1 will be described. FIG. 8 A and FIG. 8B show an example of the operation and the usage mode of the ophthalmic observation apparatus 1. While the present example describes an application to the corneal incision phase and the anterior capsulotomy (CCC) phase conducted in cataract surgery, substantially the same or a similar operation and substantially the same or a similar usage mode as those in the present example can also be implemented for applications to other phases of cataract surgery or applications to other medical practices (surgery, therapy, treatment, diagnosis, etc.).

### (S1: Commence generation and display of live image)

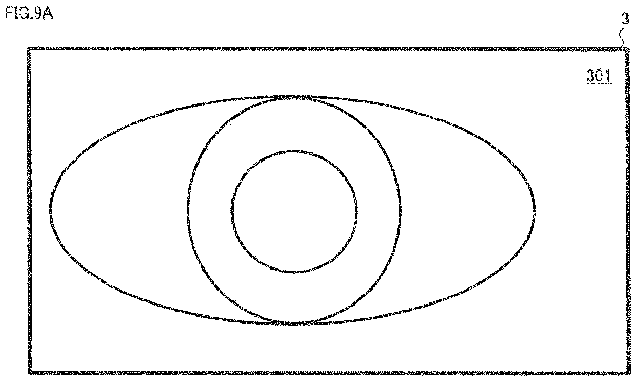

To begin with, the user performs a predetermined operation using the operation device 2 to cause the ophthalmic observation apparatus 1 to start generating and displaying a live image of the subject's eye (the anterior eye segment thereof). More specifically, the surgical microscope 10 illuminates the subject's eye by the illumination optical system 30, and at the same time (in parallel, simultaneously) generates digital image data (moving image, video) of the subject's eye by the image sensors 62L and 62R. The generated moving image (the live image 301) is displayed in real time on the display device 3 (see FIG. 9A). In other words, a moving image acquired by the surgical microscope 10 is displayed on the display device 3 as a live image (as an observation image). The user can conduct surgery while observing the live image.

### (S2: Select cataract surgery mode)

The ophthalmic observation apparatus 1 displays, on the display device 3, a screen used for selection (designation) of operation modes, for example, in response to an instruction issued by the user. Options of the operation modes may include, for example, a cataract surgery mode, a vitreoretinal surgery mode, and so forth. The user selects a desired operation mode using the operation device 2. In the present example, the cataract surgery mode is selected.

### (S3: Move to corneal incision phase)

The cataract surgery has started, and the treatment phase moves to a corneal incision.

### (S4: Detect image of predetermined site from live image)

Next, the analyzing processor 211 detects an image of the predetermined site of the subject's eye from the live image (from a frame(s) of the live image) the generation of which has started in the step S1. The site to be detected for the corneal incision may be, for example, the pupil edge or the corneal ring.

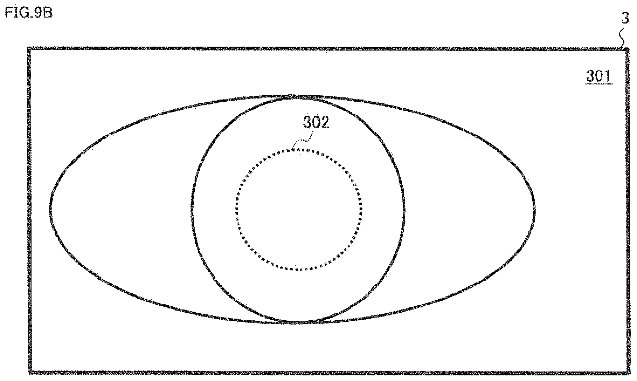

In order to detect an image of the predetermined site, in some aspects, the analyzing processor 211 first applies image processing, such as binarization, edge detection, segmentation, or other methods, to a frame of the live image 301. In the example shown in FIG. 9B, the pupil edge image 302 is detected from the live image 301.

### (S5: Determine target position for corneal incision)

Next, the target position determining processor 212 determines a corneal incision target position in the live image based at least on the image of the predetermined site (the pupil edge) detected in the step S4. The target position determining processor 212 performs, for example, any of the processing examples described above.

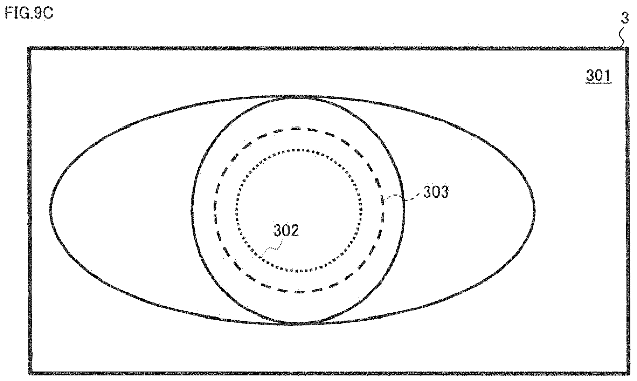

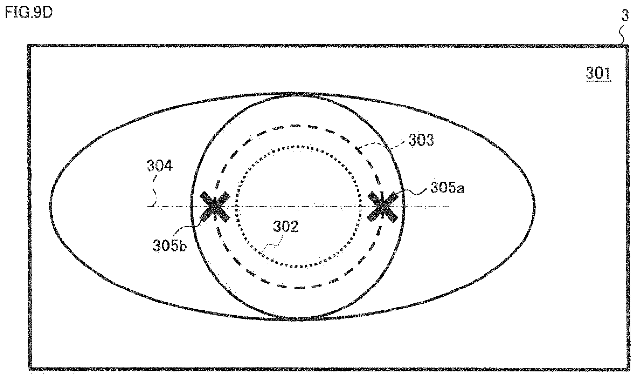

In the example shown in FIG. 9C, the target position determining processor 212 first identifies the location (position, site, area) 303 that is away from the pupil edge image 302 detected in the step S4 in an outward direction by a predetermined distance. Next, the target position determining processor 212 finds the steepest meridian 304 of the cornea of the subject's eye from the measurement data 203 (e.g., corneal shape data, corneal aberration data, etc.) acquired in advance (see FIG. 9D). The target position determining processor 212 determines, as the corneal incision target position, the position(s) at which the steepest meridian 304 and the position 303, which is the position away from the pupil edge image 302 in the outward direction by the predetermined distance, intersect each other. In the example shown in FIG. 9D, two corneal incision target positions 305a and 305b are determined.

### (S6: Display target position information for corneal incision on live image)

Next, the controller 200 displays target position information that represents the corneal incision target position determined in the step S5 on the live image of the subject's eye.

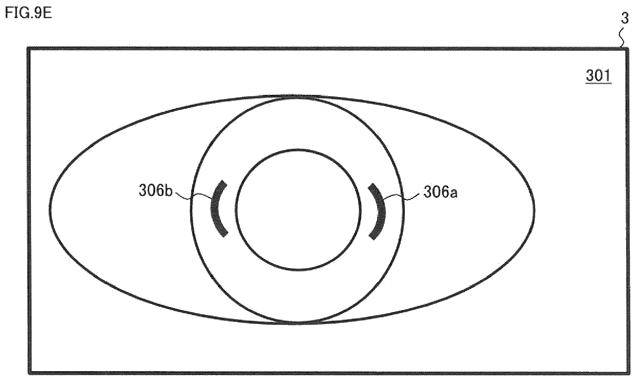

In the example shown in FIG. 9E, the two pieces of target position information 306a and 306b are displayed on the live image 301. The two pieces of target position information 306a and 306b represent the two corneal incision target positions 305a and 305b determined in the example shown in FIG. 9D, respectively. The two pieces of target position information 306a and 306b represent the positions (locations, areas) in which incisions should be made in the corneal incision. Each of the two pieces of target position information 306a and 306b is an arc-shaped indicator image. The aspect (e.g., length, radius of curvature, thickness, etc.) of the arc-shaped indicator image may be determined in advance, and/or may be freely changed according to user's instructions. This allows the user to easily recognize the position (location, area, part) in the cornea to be incised. In addition, the present example can enhance the facilitation of the steepest meridian incision for the purpose of reducing postoperative corneal astigmatism.

### (S7: Move to anterior capsulotomy?)

The series of the steps S4 to S6 is repeated at the earliest until the corneal incision is completed. Typically, the series of the steps S4 to S6 is repeatedly performed until the commencement of an ophthalmic viscosurgical device injection or the commencement of an anterior capsulotomy (CCC). The series of the steps S4 to S6 is sequentially applied to the frames that are sequentially acquired as the live image 301. For example, the series of the steps S4 to S6 may be applied to individual frames acquired as the live image 301 (time-series images). Alternatively, the series of the steps S4 to S6 may be applied to one or more frames selected from all the frames acquired as the live image 301. The process of selecting frames may include, for example, thinning of selecting frames at intervals of a predetermined number of frames. Such repetitive processing allows the user to easily recognize in real time the position (location, area, part) in the cornea to be incised, even when the corneal incision target position changes due to the movement of the subject's eye.

Corneal incision target position information (e.g., the arc-shaped indicator image(s)) may be displayed at any stage after the corneal incision is made. For example, the corneal incision target position information may be displayed in response to an instruction issued by the user. This allows the user to confirm the target position for corneal incision at a desired timing. For example, there is a problem that side ports are so small that they are easy to be lost during surgery; however, according to the present example, the user is able to confirm and know the position of the side ports at any time. It should be noted that when the position represented by the corneal incision target position information is actually incised, the controller 200 may store the coordinate information of the position represented by this corneal incision target position information in the memory 202 or another storage device. On the other hand, when a position different from the position represented by the corneal incision target position information is actually incised, the controller 200 may store, in the memory 202 or another storage device, the coordinate information of the position where the incision has actually been made. Here, together with the coordinate information of the actually incised position, the controller 200 may further store the coordinate information of the position represented by the corneal incision target position information in the memory 202 or another storage device.

### (S8: Detect image of predetermined site from live image)

Upon the commencement of the anterior capsulotomy (CCC) phase, the analyzing processor 211 detects an image of the predetermined site of the subject's eye from the live image (from a frame(s) of the live image) whose generation has started in the step S1. The site to be detected for the anterior capsulotomy may be, for example, the pupil edge or the corneal ring.

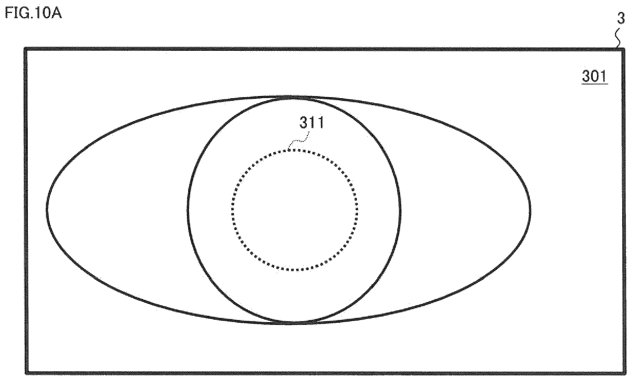

In order to detect an image of the predetermined site, in some aspects, the analyzing processor 211 first applies image processing, such as binarization, edge detection, segmentation, or other methods, to a frame of the live image 301. In the example shown in FIG. 10A, the pupil edge image 311 is detected from the live image 301 as in the corneal incision phase.

### (S9: Determine target position for anterior capsulotomy)

Next, the target position determining processor 212 determines the anterior capsulotomy target position in the live image based at least on the image of the predetermined site (pupil edge) detected in the step S8. The target position determining processor 212 performs, for example, any of the processing examples described above.

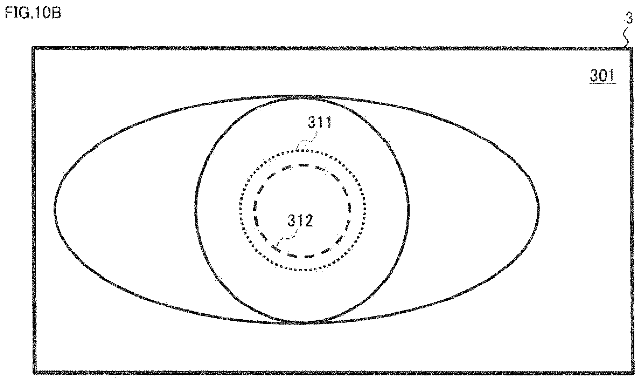

In the example shown in FIG. 10B, the target position determining processor 212 identifies the position that is away from the pupil edge image 311 detected in the step S8 in an inward direction by a predetermined distance. Then, the target position determining processor 212 sets the identified position as the anterior capsulotomy target position 312.

### (S10: Display target position information for anterior capsulotomy on live image)

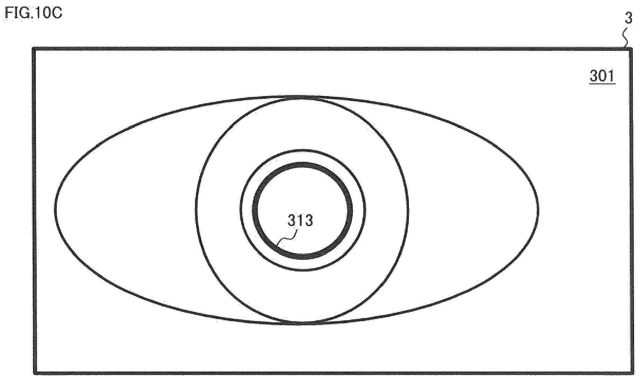

Next, the controller 200 displays target position information that represents the anterior capsulotomy target position determined in the step S9 on the live image of the subject's eye. In the example shown in FIG. 10C, the target position information 313 indicating the anterior capsulotomy target position 312 determined in the example shown in FIG. 10B, is displayed on the live image 301. The target position information 313 is a circular indicator image (instead, it may be an arc-shaped indicator image), which prompts the user to perform the anterior capsulotomy along a part of this indicator image. The aspect of the circular indicator image may be determined in advance, and/or may be freely changed according to user's instructions. This allows the user to easily perceive the position of the anterior lens capsule to be incised.

### (S11: Move to next treatment?)

The series of the steps S8 to S10 is repeated at the earliest until the anterior capsulotomy is completed. Typically, the series of the steps S8 to S10 is repeatedly performed until the commencement of phacoemulsification and aspiration. The series of the steps S8 to S10 is sequentially applied to the frames that are sequentially acquired as the live image 301. For example, the series of the steps S8 to S10 may be applied to individual frames acquired as the live image 301 (time-series images). Alternatively, the series of the steps S8 to S10 may be applied to one or more frames selected from all the frames acquired as the live image 301. The process of selecting frames may include, for example, thinning of selecting frames at intervals of a predetermined number of frames. Such repetitive processing allows the user to easily recognize in real time the position (location, area, part) in the anterior lens capsule to be incised, even when the anterior capsulotomy target position changes due to the movement of the subject's eye.

Anterior capsulotomy target position information (e.g., the circular indicator image) may be displayed at any stage after the incision is made on the anterior lens capsule. For example, the anterior capsulotomy target position information may be displayed in response to an instruction issued by the user. This allows the user to confirm the target position for anterior capsulotomy at a desired timing. When the position represented by the anterior capsulotomy target position information is actually incised, the controller 200 may store the coordinate information of the position represented by this anterior capsulotomy target position information in the memory 202 or another storage device. On the other hand, when a position different from the position represented by the anterior capsulotomy target position information is actually incised, the controller 200 may store, in the memory 202 or another storage device, the coordinate information of the position where the incision has actually been made. Here, together with the coordinate information of the actually incised position, the controller 200 may further store the coordinate information of the position represented by the anterior capsulotomy target position information in the memory 202 or another storage device.

### (S12: Apply other treatments)

After the completion of the anterior capsulotomy, the user sequentially conducts subsequent treatments such as phacoemulsification and aspiration, IOL insertion, ophthalmic viscosurgical device removal, and incision closure. This completes the cataract surgery (End).

Some other aspect examples are briefly described below. In vitreoretinal surgery, for example, the analyzing processor 211 first applies image processing (e.g., binarization) to an observation image to detect an image of the corneal ring. Next, the target position determining processor 212 identifies a position that is a predetermined distance (e.g., 3 to 4 millimeters) outward from the detected image of the corneal ring as a target position. Then, the controller 200 displays an indicator (e.g., an indicator having a circular shape) at the target position.

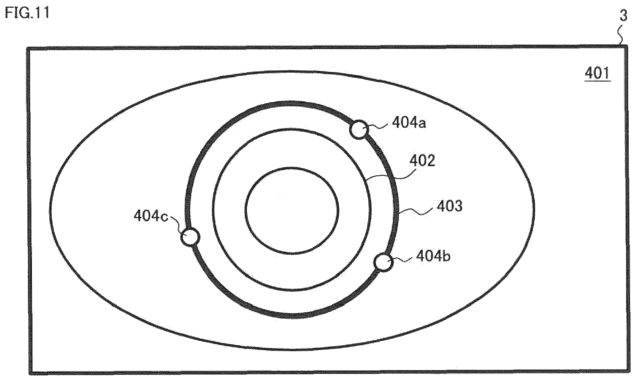

FIG. 11 shows an example of information displayed for vitreoretinal surgery. In the present example, along with the live image 401 acquired by the surgical microscope 10, the circular indicator image 403 that represents the position that is the predetermined distance (e.g., 3 to 4 millimeters) outward from the image of the corneal ring 402 is displayed. The user can create a port by making a scleral incision (conjunctival incision) in vitreoretinal surgery while referring to the indicator image 403. The port provides a pathway for inserting a light guide (illumination) and various kinds of instruments into the eye. FIG. 11 presents the three ports 404a, 404b, and 404c formed on the indicator image 403. The ophthalmic observation apparatus 1 may record the coordinate information of each of the ports formed, and furthermore, may display indicator images respectively indicating the positions of the ports on the live image in response to a request from the user, for example.

As described thus far, ophthalmic surgeries not according to the claimed invention are typically conducted by inserting an instrument(s) into the eye. The present disclosure may be configured to, for example, display the optimal treatment position (e.g., instrument insertion position, incision position, perforation position, etc.) on a live image. For example, by displaying an indicator (e.g., an image) that represents a treatment target position during surgery, the marking of the treatment target position can be made within the user's field of view, thereby facilitating the surgery.

Furthermore, the present disclosure can execute tracking of the treatment target position by sequentially applying processing to a plurality of frames that are sequentially acquired as a live image. In addition, the present disclosure can present the treatment position on the live image after the corresponding treatment has actually been executed.

Although the present disclosure describes several applications to cataract surgery (e.g., at the time of corneal incision, at the time of anterior capsulotomy, etc.) and vitreoretinal surgery, those skilled in the art will understand that possible applications of the present disclosure are not limited thereto.

Note that some aspects can, at the time of corneal incision for cataract surgery, display a figure (e.g., an arc-shaped line) that serves as an incision target based on a feature of the anterior eye segment (e.g., the corneal ring, the pupil, the size of the corneal ring, the size of the pupil, etc.) and measurement data of the subject's eye. The size and the shape of this figure may be changeable. It should also be noted that marking processing for the corneal incision can be made prior to or during the surgery by carrying out the following processes: acquiring corneal data by means of a wavefront sensor, a corneal topographer, or an anterior eye segment OCT; identifying a position where the effect on corneal astigmatism is small; and finding a feature position (e.g., the pupil edge, the pupil center) from a live image.

Some aspects can, at the time of anterior capsulotomy for cataract surgery, display a figure (e.g., a circle of a diameter of 6 millimeters) determined from a feature of the anterior eye segment (e.g., the corneal ring, the pupil, the size of the corneal ring, the size of the pupil, etc.) as an incision target. The size and the shape of this figure may be changeable. Note that marking processing for the anterior capsulotomy can be made prior to or during the surgery by carrying out the following processes: acquiring a predetermined kind of data (e.g., a corneal ring diameter, a pupil diameter); determining the size of the indicator image (figure) to be displayed; detecting a feature part (e.g., the corneal ring, the pupil edge) from a live image; and displaying an indicator image having the size determined.

Some aspects can, at the time of vitreoretinal surgery, display a figure (e.g., a circular image) that serves as an instrument insertion target (e.g., perforation target, port formation target) at a position away from the corneal ring in an outward direction by a predetermined distance (e.g., 3 to 4 millimeters). The size and the shape of this figure may be changeable.

In surgeries, treatments, therapies, examinations, and so forth other than the above-described kinds of surgeries and the like, target position information indicating a position to which a predetermined treatment should be applied, can be displayed together with a live image.

This makes it possible to achieve easier treatment, shorter treatment, fewer errors in treatment, and less burden on patients during surgery, treatment, and examination.

### < Method of controlling ophthalmic observation apparatus >

Some embodiment examples (e.g., the ophthalmic observation apparatus 1 described above) provide a method of controlling an ophthalmic observation apparatus. It is possible to combine any items or matters relating to the ophthalmic observation apparatus 1 of the above embodiment examples with the example of the method described below.

An ophthalmic observation apparatus controlled by the method of an aspect example includes a processor (e.g., the controller 200 and the data processor 210) and generates a moving image of a subject's eye (the surgical microscope 10). The method of the present aspect example first causes the processor to analyze a still image included in the moving image to detect an image of a predetermined site of the subject's eye. Further, the method of the present aspect example causes the processor to determine a target position for a predetermined treatment based at least on the image of the predetermined site detected from the still image. In addition, the method of the present aspect example causes the processor to display the moving image on a display device and also display target position information that represents the determined target position on the display device.

The method of the present aspect example is capable of achieving the same actions and effects as those of the ophthalmic observation apparatus 1 of the above embodiment examples. In addition, by combining any of the matters and items relating to the ophthalmic observation apparatus 1 of the above embodiment examples with the method of the present aspect example, the resulting method becomes capable of achieving the actions and effects corresponding to the combined matters and/or items.

### < Program >

Some embodiment examples provide a program causing a computer to execute the method of the aspect example described above. It is possible to combine any of the matters and items relating to the ophthalmic observation apparatus 1 of the above embodiment examples with such a program.

The program thus configured is capable of achieving the same actions and effects as those of the ophthalmic observation apparatus 1 of the above embodiment examples. In addition, by combining any of the matters and items relating to the ophthalmic observation apparatus 1 of the above embodiment examples with the program, the resulting program is capable of achieving the actions and effects corresponding to the combined matters and/or items.

### < Recording medium >

Some embodiment examples according to the claimed invention provide a computer-readable non-transitory recording medium storing the program described above. It is possible to combine any of the matters and items relating to the ophthalmic observation apparatus 1 of the above embodiment examples with such a recording medium. The non-transitory recording medium may be in any form, and examples thereof include magnetic disks, optical disks, magneto-optical disks, and semiconductor memories.

The recording medium thus configured is capable of achieving the same actions and effects as those of the ophthalmic observation apparatus 1 of the above embodiment examples. In addition, by combining any of the matters and items relating to the ophthalmic observation apparatus 1 of the above embodiment examples with the recording medium, the resulting recording medium is capable of achieving the actions and effects corresponding to the combined matters and/or items.

## Claims

1. An ophthalmic observation apparatus (1) for observing a subject's eye (E), comprising:
a moving image generating unit (30, 40) configured to generate a moving image by photographing the subject's eye (E);
an analyzing processor (211) configured to analyze a still image included in the moving image to detect an image of a predetermined site of the subject's eye (E);
a measuring unit (220) configured to acquire measurement data of the subject's eye (E);
a target position determining processor (212C) configured to determine a target position for a predetermined treatment based at least on the image of the predetermined site detected by the analyzing processor (211) and the measurement data acquired by the measuring unit (220); and
a display controller (200) configured to display, on a display device (3), the moving image and target position information that represents the target position, wherein
the target position for the predetermined treatment is a target incision position for ocular incision,
the measurement unit (220) includes at least one of a wavefront sensor configured to acquire corneal aberration data of the subject's eye (E), a corneal topographer configured to acquire corneal shape data of the subject's eye (E), and an anterior eye segment OCT apparatus configured to acquire corneal shape data of the subject's eye (E), and the measurement unit (220) is configured to acquire the measurement data including at least one of the corneal aberration data acquired by the wavefront sensor, the corneal shape data acquired by the corneal topographer, and the corneal shape data acquired by the anterior eye segment OCT apparatus,
the analyzing processor (211) is configured to detect an image of a cornea of the subject's eye (E) as the image of the predetermined site, and
the target position determining processor (212C) is configured to identify, as the target incision position, a position corresponding to a direction of a steepest meridian in the cornea based on the image of the cornea and on data that represents a steepest meridian position either included in the measurement data or derived from the measurement data,
wherein the target position determining processor (212C) includes an assessing processor (213) configured to assess influence of the ocular incision on corneal astigmatism of the subject's eye (E) based on the measurement data acquired by the measuring unit (220), and
the target position determining processor (212C) is configured to determine the target incision position based at least on the image of the cornea detected by the analyzing processor (211) and a result obtained by the assessing processor (213),
and wherein the assessing processor (213) is configured to perform
determination of a state of the cornea before the ocular incision including at least one of the direction of the steepest meridian, a refractive power in the direction of the steepest meridian, a direction of a flat meridian, refractive power in the direction of the flat meridian, and a distribution of refractive powers, from the at least one of the corneal aberration data acquired by the wavefront sensor, the corneal shape data acquired by the corneal topographer, and the corneal shape data acquired by the anterior eye segment OCT apparatus, and
simulation of a state of the cornea after the ocular incision based at least on the state of the cornea before the ocular incision.

2. The ophthalmic observation apparatus according to claim 1, wherein
the assessing processor (213) is configured to perform the simulation by means of an inference model constructed by supervised learning with training data that includes states of corneas before ocular incisions, attributes of the ocular incisions, and states of the corneas after the ocular incisions.

3. A method of controlling an ophthalmic observation apparatus that includes a processor (200, 210) and a measuring unit (220) that acquires measurement data of a subject's eye (E) and that generates a moving image of a subject's eye (E), the method comprising:
causing the processor (200, 210) to analyze a still image included in the moving image to detect an image of a predetermined site of the subject's eye (E);
causing the processor (200, 210) to determine a target position for a predetermined treatment based at least on the image of the predetermined site detected and the measurement data acquired by the measurement unit (220);
causing the processor (200, 210) to display, on a display device (3), the moving image and target position information that represents the target position, wherein
the target position for the predetermined treatment is a target incision position for ocular incision,
the measurement data includes at least one of corneal aberration data and corneal shape data of the subject's eye (E),
the analysis of the still image detects an image of a cornea of the subject's eye (E) as the image of the predetermined site, and
the determination of the target position identifies, as the target incision position, a position corresponding to a direction of a steepest meridian in the cornea based on the image of the cornea and on data representing a steepest meridian position either included in the measurement data or derived from the measurement data;
causing the processor (200, 210) to assess influence of the ocular incision on corneal astigmatism of the subject's eye (E) based on the measurement data, wherein the determination of the target position determines the target incision position based at least on the image of the cornea and a result obtained by the assessing of the influence of the ocular incision on corneal astigmatism; and
causing the processor (200, 210)
to determine a state of the cornea before the ocular incision including at least one of the direction of the steepest meridian, a refractive power in the direction of the steepest meridian, a direction of a flat meridian, refractive power in the direction of the flat meridian, and a distribution of refractive powers, from the at least one of the corneal aberration data and the corneal shape data, and
to simulate of a state of the cornea after the ocular incision based at least on the state of the cornea before the ocular incision.

4. A computer-readable non-transitory recording medium storing a program configured to cause the device of claim 1 to execute the steps of the method of claim 3.

## Patentansprüche

1. Ophthalmische Beobachtungsvorrichtung (1) zum Beobachten eines Auges (E) einer Person, die Folgendes umfasst:
eine Bewegtbilderzeugungseinheit (30, 40), die so konfiguriert ist, dass sie durch Fotografieren des Auges (E) der Person ein Bewegtbild erzeugt,
einen Analyseprozessor (211), der so konfiguriert ist, dass er ein in dem Bewegtbild enthaltenes Standbild analysiert, um ein Bild von einer vorgegebenen Lokalisation am Auge (E) der Person zu erkennen,
eine Messeinheit (220), die so konfiguriert ist, dass sie Messdaten zum Auge (E) der Person erfasst,
einen Zielpositionsbestimmungsprozessor (212C), der so konfiguriert ist, dass er zumindest auf der Grundlage des von dem Analyseprozessor (211) erkannten Bildes von der vorgegebenen Lokalisation und der von der Messeinheit (220) erfassten Messdaten eine Zielposition für eine vorgegebene Behandlung bestimmt, und
eine Anzeigesteuerung (200), die so konfiguriert ist, dass sie auf einer Anzeigeeinrichtung (3) das Bewegtbild und Zielpositionsinformationen anzeigt, die die Zielposition darstellen, wobei
es sich bei der Zielposition für die vorgegebene Behandlung um eine Einschnitt-Zielposition für einen okularen Einschnitt handelt,
die Messeinheit (220) Folgendes aufweist: einen Wellenfrontsensor, der so konfiguriert ist, dass er Daten zu Hornhautabweichungen am Auge (E) der Person erfasst, oder/und einen Hornhaut-Topographen, der so konfiguriert ist, dass er Daten zur Form der Hornhaut des Auges (E) der Person erfasst, oder/und eine OCT-Vorrichtung für den vorderen Augenabschnitt, die so konfiguriert ist, dass sie Daten zur Form der Hornhaut des Auges (E) der Person erfasst, und die Messeinheit (220) so konfiguriert ist, dass sie die Messdaten einschließlich der von dem Wellenfrontsensor erfassten Daten zu Hornhautabweichungen oder/und der von dem Hornhaut-Topographen erfassten Daten zur Form der Hornhaut oder/und der von der OCT-Vorrichtung für den vorderen Augenabschnitt erfassten Daten zur Form der Hornhaut erfasst,
der Analyseprozessor (211) so konfiguriert ist, dass er ein Bild von einer Hornhaut des Auges (E) der Person als Bild von der vorgegebenen Lokalisation erkennt, und
der Zielpositionsbestimmungsprozessor (212C) so konfiguriert ist, dass er auf der Grundlage des Bildes von der Hornhaut und von Daten, die eine Position eines steilsten Meridians darstellen und entweder in den Messdaten enthalten oder aus diesen abgeleitet sind, eine Position, die einer Richtung eines steilsten Meridians in der Hornhaut entspricht, als Einschnitt-Zielposition ermittelt,
wobei der Zielpositionsbestimmungsprozessor (212C) einen Beurteilungsprozessor (213) aufweist, der so konfiguriert ist, dass er auf der Grundlage der von der Messeinheit (220) erfassten Messdaten einen Einfluss des okularen Einschnitts auf einen Hornhautastigmatismus des Auges (E) der Person beurteilt, und
der Zielpositionsbestimmungsprozessor (212C) so konfiguriert ist, dass er die Einschnitt-Zielposition auf der Grundlage des von dem Analyseprozessor (211) erkannten Bildes von der Hornhaut oder/und eines von dem Beurteilungsprozessor (213) gewonnenen Ergebnisses bestimmt,
und wobei der Beurteilungsprozessor (213) so konfiguriert ist, dass er Folgendes durchführt:
Bestimmung eines Zustands der Hornhaut vor dem okularen Einschnitt, einschließlich der Richtung des steilsten Meridians oder/und einer Brechkraft in der Richtung des steilsten Meridians oder/und einer Richtung eines flachen Meridians oder/und einer Brechkraft in der Richtung des flachen Meridians oder/und einer Verteilung der Brechkräfte, anhand der von dem Wellenfrontsensor erfassten Daten zu Hornhautabweichungen oder/und der von dem Hornhaut-Topographen erfassten Daten zur Form der Hornhaut oder/und der von der OCT-Vorrichtung für den vorderen Augenabschnitt erfassten Daten zur Form der Hornhaut, und
Simulation eines Zustands der Hornhaut nach dem okularen Einschnitt zumindest auf der Grundlage des Zustands der Hornhaut vor dem okularen Einschnitt.

2. Ophthalmische Beobachtungsvorrichtung nach Anspruch 1, wobei
der Beurteilungsprozessor (213) so konfiguriert ist, dass er die Simulation mithilfe eines Inferenzmodells durchführt, das durch überwachtes Lernen mit Trainingsdaten erstellt wird, die Zustände von Hornhäuten vor okularen Einschnitten, Attribute der okularen Einschnitte und Zustände der Hornhäute nach den okularen Einschnitten umfassen.

3. Verfahren zum Steuern einer ophthalmischen Beobachtungsvorrichtung, die einen Prozessor (200, 210) und eine Messeinheit (220) aufweist, welche Messdaten zu einem Auge (E) einer Person erfasst, und ein Bewegtbild von einem Auge (E) einer Person erzeugt, wobei das Verfahren Folgendes umfasst:
Veranlassen, dass der Prozessor (200, 210) ein in dem Bewegtbild enthaltenes Standbild analysiert, um ein Bild von einer vorgegebenen Lokalisation am Auge (E) der Person zu erkennen,
Veranlassen, dass der Prozessor (200, 210) zumindest auf der Grundlage des erkannten Bildes von der vorgegebenen Lokalisation und der von der Messeinheit (220) erfassten Messdaten eine Zielposition für eine vorgegebene Behandlung bestimmt, und
Veranlassen, dass der Prozessor (200, 210) auf einer Anzeigeeinrichtung (3) das Bewegtbild und Zielpositionsinformationen anzeigt, die die Zielposition darstellen, wobei
es sich bei der Zielposition für die vorgegebene Behandlung um eine Einschnitt-Zielposition für einen okularen Einschnitt handelt,
die Messdaten Daten zu Hornhautabweichungen am Auge (E) der Person oder/und Daten zur Form der Hornhaut des Auges (E) der Person umfassen,
bei der Analyse des Standbildes ein Bild von einer Hornhaut des Auges (E) der Person als Bild von der vorgegebenen Lokalisation erkannt wird und
bei der Bestimmung der Zielposition eine Position, die einer Richtung eines steilsten Meridians in der Hornhaut entspricht, auf der Grundlage des Bildes von der Hornhaut und von Daten, die eine Position eines steilsten Meridians darstellen und entweder in den Messdaten enthalten sind oder aus diesen abgeleitet werden, als Einschnitt-Zielposition ermittelt wird,
Veranlassen, dass der Prozessor (200, 210) auf der Grundlage der Messdaten einen Einfluss des okularen Einschnitts auf den Hornhautastigmatismus des Auges (E) der Person beurteilt, wobei bei der Bestimmung der Zielposition die Einschnitt-Zielposition zumindest auf der Grundlage des Bildes von der Hornhaut und eines durch das Beurteilen des Einflusses des okularen Einschnitts auf den Hornhautastigmatismus gewonnenen Ergebnisses bestimmt wird, und
Veranlassen, dass der Prozessor (200, 210)
anhand der Daten zu Hornhautabweichungen oder/und der Daten zur Form der Hornhaut einen Zustand der Hornhaut vor dem okularen Einschnitt, einschließlich der Richtung des steilsten Meridians oder/und einer Brechkraft in der Richtung des steilsten Meridians oder/und einer Richtung eines flachen Meridians oder/und einer Brechkraft in der Richtung des flachen Meridians oder/und einer Verteilung der Brechkräfte, bestimmt, und
zumindest auf der Grundlage des Zustands der Hornhaut vor dem okularen Einschnitt einen Zustand der Hornhaut nach dem okularen Einschnitt simuliert.

4. Computerlesbares, nichtflüchtiges Aufzeichnungsmedium, auf dem ein Programm gespeichert ist, das so konfiguriert ist, dass es die Vorrichtung nach Anspruch 1 dazu veranlasst, die Schritte des Verfahrens nach Anspruch 3 auszuführen.

## Revendications

1. Dispositif d'observation ophtalmologique (1) pour observer l'œil d'un sujet (E), comprenant :
une unité de génération d'image animée (30, 40) configurée pour générer une image animée en photographiant l'œil du sujet (E) ;
un processeur d'analyse (211) configuré pour analyser une image fixe incluse dans l'image animée afin de détecter une image d'un site prédéterminé de l'œil du sujet (E) ;
une unité de mesure (220) configurée pour acquérir des données de mesure de l'œil du sujet (E) ;
un processeur de détermination de position cible (212C) configuré pour déterminer une position cible pour un traitement prédéterminé sur la base d'au moins l'image du site prédéterminé détectée par le processeur d'analyse (211) et les données de mesure acquises par l'unité de mesure (220) ; et
un contrôleur d'affichage (200) configuré pour afficher, sur un dispositif d'affichage (3), l'image animée et les informations de position cible qui représentent la position cible,
la position cible pour le traitement prédéterminé étant une position d'incision cible pour l'incision oculaire,
l'unité de mesure (220) comprenant au moins un capteur de front d'onde configuré pour acquérir des données d'aberration cornéenne de l'œil du sujet (E), un topographe cornéen configuré pour acquérir des données de forme cornéenne de l'œil du sujet (E), et un appareil OCT de segment d'œil antérieur configuré pour acquérir des données de forme cornéenne de l'œil du sujet (E), et l'unité de mesure (220) étant configurée pour acquérir les données de mesure comprenant au moins une des données d'aberration cornéenne acquises par le capteur de front d'onde, des données de forme cornéenne acquises par le topographe cornéen et des données de forme cornéenne acquises par l'appareil OCT de segment oculaire antérieur,
le processeur d'analyse (211) étant configuré pour détecter une image de la cornée de l'œil du sujet (E) comme image du site prédéterminé, et
le processeur de détermination de position cible (212C) étant configuré pour identifier, comme position d'incision cible, une position correspondant à la direction d'un méridien le plus abrupt dans la cornée sur la base de l'image de la cornée et des données qui représentent une position méridienne la plus abrupt, soit incluses dans les données de mesure, soit dérivées des données de mesure,
le processeur de détermination de position cible (212C) comprenant un processeur d'évaluation (213) configuré pour évaluer l'influence de l'incision oculaire sur l'astigmatisme cornéen de l'œil du sujet (E) sur la base des données de mesure acquises par l'unité de mesure (220), et
le processeur de détermination de position cible (212C) étant configuré pour déterminer la position d'incision cible sur la base au moins de l'image de la cornée détectée par le processeur d'analyse (211) et d'un résultat obtenu par le processeur d'évaluation (213).
et le processeur d'évaluation (213) étant configuré pour effectuer
la détermination d'un état de la cornée avant l'incision oculaire comprenant au moins l'un des éléments suivants : la direction du méridien le plus abrupt, une puissance de réfraction dans la direction du méridien le plus abrupt, une direction d'un méridien plat, une puissance de réfraction dans la direction du méridien plat, et une répartition des puissances de réfraction, à partir de l'au moins une des données d'aberration cornéenne acquises par le capteur de front d'onde, des données de forme cornéenne acquises par le topographe cornéen, et des données de forme cornéenne acquises par l'appareil OCT de segment oculaire antérieur, et
la simulation d'un état de la cornée après l'incision oculaire basée au moins sur l'état de la cornée avant l'incision oculaire.

2. Dispositif d'observation ophtalmologique selon la revendication 1, dans lequel
le processeur d'évaluation (213) est configuré pour effectuer la simulation au moyen d'un modèle d'inférence construit par apprentissage supervisé avec des données d'apprentissage qui comprennent les états des cornées avant les incisions oculaires, les attributs des incisions oculaires et les états des cornées après les incisions oculaires.

3. Procédé de commande d'un dispositif d'observation ophtalmologique qui comprend un processeur (200, 210) et une unité de mesure (220) qui acquiert des données de mesure de l'œil d'un sujet (E) et qui génère une image animée de l'œil d'un sujet (E), le procédé comprenant les étapes consistant à :
amener le processeur (200, 210) à analyser une image fixe incluse dans l'image animée afin de détecter une image d'un site prédéterminé de l'œil du sujet (E) ;
amener le processeur (200, 210) à déterminer une position cible pour un traitement prédéterminé sur la base au moins de l'image du site prédéterminé détectée et des données de mesure acquises par l'unité de mesure (220) ;
amener le processeur (200, 210) à afficher, sur un dispositif d'affichage (3), l'image animée et les informations de position cible qui représentent la position cible,
la position cible pour le traitement prédéterminé étant une position d'incision cible pour l'incision oculaire,
les données de mesure comprenant au moins des données d'aberration cornéenne et des données de forme cornéenne de l'œil du sujet (E),
l'analyse de l'image fixe détectant une image d'une cornée de l'œil du sujet (E) comme image du site prédéterminé, et
la détermination de la position cible identifiant, en tant que position cible de l'incision, une position correspondant à la direction d'un méridien le plus abrupt de la cornée sur la base de l'image de la cornée et de données représentant une position méridienne la plus abrupte, soit incluses dans les données de mesure, soit dérivées des données de mesure;
amener le processeur (200, 210) à évaluer l'influence de l'incision oculaire sur l'astigmatisme cornéen de l'œil du sujet (E) sur la base des données de mesure, la détermination de la position cible déterminant la position cible de l'incision sur la base au moins de l'image de la cornée et d'un résultat obtenu par l'évaluation de l'influence de l'incision oculaire sur l'astigmatisme cornéen ; et
amener le processeur (200, 210) à
déterminer un état de la cornée avant l'incision oculaire comprenant au moins une des directions du méridien le plus abrupt, une puissance de réfraction dans la direction du méridien le plus abrupt, une direction d'un méridien plat, une puissance de réfraction dans la direction du méridien plat, et une répartition des puissances de réfraction, à partir d'au moins l'une des données d'aberration cornéenne et des données de forme cornéenne, et
simuler un état de la cornée après l'incision oculaire en se basant au moins sur l'état de la cornée avant l'incision oculaire.

4. Support d'enregistrement non transitoire lisible par ordinateur stockant un programme conçu pour amener le dispositif selon la revendication 1 à exécuter les étapes du procédé selon la revendication 3.
